# EUROPEAN PATENT APPLICATION

(11) **EP 1 816 135 A1**
(43) Date of publication of application: **08.08.2007**
(21) Application number: 06001862.9
(22) Date of filing: 30.01.2006
(51) Int. Cl.: C07H 19/173, C07H 19/19

(54) **Production method of fluorinated purine nucleoside derivative, intermediate therefor and production method thereof**

(71) Applicant: Ajinomoto Co., Inc., Tokyo 104-8315 (JP)
(72) Inventor: Tori, Takayoshi, Kawasaki-shi Kanagawa 210-8681 (JP); Onishi, Tomoyuki, Kawasaki-shi Kanagawa 210-8681 (JP); Izawa, Kunisuke, Kawasaki-shi Kanagawa 210-8681 (JP)
(74) Representative: Strehl Schübel-Hopf & Partner

(57) **Abstract**

Purine nucleosides which are fluorinated at the 3'-position (preferably the α-position), may be economically and efficiently produced by fluorinating a novel purine nucleoside derivative (1) in which the hydroxyl group at the 5'-position is protected to obtain a novel purine nucleoside derivative (2) in a high yield. The derivative (2) is subjected to desulfurization, deprotection of R¹ and, as necessary protection, deprotection, or modification of nucleic acid base moiety, to obtain the desired purine nucleoside (3). 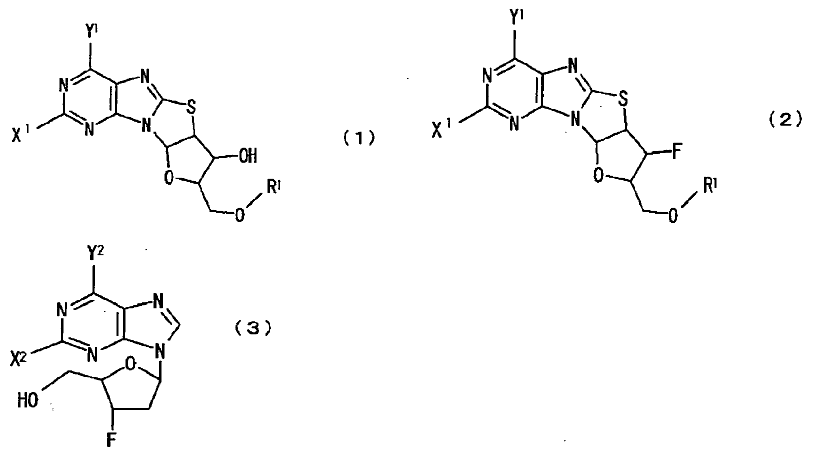 wherein each symbol is as defined in the specification.

## Description

### TECHNICAL FIELD OF THE INVENTION

The present invention relates to methods of producing purine nucleoside derivatives which are fluorinated at the 3'-position (preferably the α-position). More particularly, the present invention relates to intermediate compounds which are useful for the production of such derivatives and production methods thereof.

### BACKGROUND OF THE INVENTION

Purine nucleoside derivatives which are fluorinated at the 3'-position (preferably the α-position) have been drawing attention since they exhibit an antitumor or antiviral effect. For example, 2',3'-dideoxy-3'-fluoroadenosine is disclosed in the specification of East German Patent No. 158903; 2',3'-dideoxy-3'-fluoroguanosine is disclosed in the specification of East German Patent No. 209197; and 2',3'-dideoxy-3'-fluoroinosine is disclosed in J. Med. Chem., vol. 32, p. 1743 (1989).

As a synthesis method of a nucleoside derivative which is fluorinated at the 3'-α-position, for example, a method is known which includes obtaining a derivative which is fluorinated at the 3'-α-position, which has been sterically inverted from a derivative having a hydroxyl group at the 3'-β-position by SN₂ substitution reaction (J. Med. Chem., vol. 31, p. 2040 (1988).

According to this method, however, the yield is as low as 35% since side reactions such as elimination reaction and the like preferentially occur even when diethylaminosulfur trifluoride (DAST), known as the most efficient fluorinating agent, is used in the fluorination step. Therefore, the method is not satisfactory.

In addition, a method of obtaining a nucleoside derivative which is fluorinated at the 3'-α-position, which includes synthesizing a sugar which is fluorinated at the corresponding 3-α-position and coupling the sugar with a nucleic acid base, is also known. With regard to this method, the synthesis method of a sugar which is fluorinated at the 3-α-position is disclosed in Liebigs Ann. Chem., 1137 (1990) and Bioorg. Med. Chem. Lett., vol. 13, p. 817 (2003), and the method of coupling the obtained sugar which is fluorinated at the 3-α-position with a nucleic acid base is disclosed in Carbohydr. Res., vol. 328, p. 49 (2000) and Tetrahedron Lett., vol. 44, p. 2899 (2003).

In the above-mentioned Liebigs Ann. Chem., 1137 (1990), 2-deoxyribose as a starting material is converted into a sugar having a hydroxyl group at the 3-β-position via methylation of anomer hydroxyl group, introduction of a protecting group at the 5-position, oxidization of a hydroxyl group at the 3-position, and reduction of the resulting compound. The sugar is then reacted with a fluorinating agent (DAST) to obtain a sugar which is fluorinated at the 3-α-position. According to this method, however, multiple steps are necessary for synthesizing the fluorinated sugar, and, considering the facts that the yield is low due to an elimination reaction that occurs as a side reaction in the fluorination step, and a coupling reaction with a nucleic acid base is necessary, this method is not suitable as an industrial production method.

In the above-mentioned Bioorg. Med. Chem. Lett., vol. 13, p. 817 (2003), a sugar which is fluorinated at the 3-α-position is synthesized using xylose as a starting material. According to this method, however, multiple steps are necessary for obtaining the sugar which is fluorinated at the 3-α-position, and deoxygenation of the 2-position hydroxyl group is difficult. Thus, this method, too, is not suitable as an industrial production method.

The above-mentioned Carbohydr. Res., vol. 328, p. 49 (2000) describes a method for the chemical coupling of a sugar which is fluorinated at the 3-α-position with a nucleic acid base. According to this coupling reaction, however, the α anomer and β anomer are produced at a ratio of 1:1, and a theoretical yield of more than 50% is beyond expectation. In addition, since separation of an α anomer from a β anomer (production ratio 1:1) is generally difficult, this method is not suitable as an industrial production method.

In the method described in the above-mentioned Tetrahedron Lett., vol. 44, p. 2899 (2003), a phosphonate group is introduced into the 1-position of a sugar which is fluorinated at the 3-α-position, and the sugar is then enzymatically coupled with a nucleic acid base to selectively give a β-anomer. According to this coupling method, however, obtaining the 1-position phosphonate compound, which is a substrate, from a α-methoxy compound requires three steps, and this method is not suitable as an industrial production method.

In summary, the methods described in the above-mentioned publications, Liebigs Ann. Chem., 1137 (1990), Bioorg. Med. Chem. Lett., vol. 13, p. 817 (2003), Carbohydr. Res., vol. 328, p. 49 (2000), and Tetrahedron Lett., vol. 44, p. 2899 (2003), have problems in the both steps of obtaining a sugar which is fluorinated at the 3'-α-position and coupling the sugar with a nucleic acid base.

Furthermore, a method is known which includes synthesizing a pyrimidine nucleoside derivative which is fluorinated at the 3'-α-position from thymidine as a starting material (*see* European Patent Application No. 495 224), and converting the pyrimidine nucleoside derivative which is fluorinated at the 3'-α-position to a purine nucleoside derivative which is fluorinated at the 3'-α-position by enzymatic transglycosylation (*see* U.S. Patent No. 5,637,574).

However, since this method requires a combined use of hydrogen fluoride and aluminum trifluoride which are difficult to handle in the fluorination step, a two-step enzyme reaction, and the like, it is not suitable as an industrial production method.

Furthermore, a method of synthesizing a 2,3-dideoxy-3-fluoro-β-D-ribofuranosyl type nucleoside derivative is known, which includes reacting a 3-deoxy-3-halogeno-β-D-xylofuranosyl type nucleoside derivative having a hydroxyl group or a silyloxy group (silylated hydroxyl group) at the 2'-α-position and a halogen atom other than fluorine atom at the 3'-β-position, with dialkylaminosulfur trifluoride to convert the derivative to a 2,3-dideoxy-3-fluoro-2-halogeno-β-D-arabinofuranosyl type nucleoside derivative with a halogen atom inverted to the 2'-β-position and fluorinated at the 3'-α-position, and then dehalogenating the 2'-position halogen atom (*see* Nucleosides Nucleotides & Nucleic Acids, vol. 22, p. 711 (2003). However, this method has a problem of insufficient regioselectivity in the fluorination of the 2'-position and the 3'-position during the fluorination step.

Thus, there remains a need for methods of producing purine nucleoside derivatives which are fluorinated at the 3'-position (preferably the α-position) which do not suffer from the above-mentioned drawbacks. There also remain a need for compounds which are useful in such methods and production methods thereof.

### SUMMARY OF THE INVENTION

Accordingly, it is one object of the present invention to provide novel methods for producing purine nucleoside derivatives which are fluorinated at the 3'-position.

It is another object of the present invention to provide novel methods for producing purine nucleoside derivatives which are fluorinated at the 3'-position (preferably the α-position).

It is another object of the present invention to provide novel economic and efficient methods of producing a purine nucleoside derivatives which are fluorinated at the 3'-position.

It is another object of the present invention to provide novel economic and efficient methods of producing a purine nucleoside derivatives which are fluorinated at the 3'-position (preferably the α-position).

It is another object of the present invention to provide novel methods of producing a purine nucleoside derivatives which are fluorinated at the 3'-position, which are suitable as an industrial production method.

It is another object of the present invention to provide economic and efficient methods of producing a purine nucleoside derivatives which are fluorinated at the 3'-position (preferably the α-position), which are suitable as an industrial production method.

It is another object of the present invention to provide novel intermediates which are useful in such methods.

It is another object of the present invention to provide novel methods for producing such intermediates.

It is another object of the present invention to provide novel economic and efficient methods for producing such intermediates.

It is another object of the present invention to provide novel methods for producing such intermediates, which are suitable as an industrial production method.

These and other objects, which will become apparent during the following detailed description, have been achieved by the inventors' discovery that a novel 8,2'-anhydro-3'-deoxy-3'-fluoro-8-mercapto-9-arabinofuranosyl purine derivative which is fluorinated at the 3'-position (preferably α-position) can be obtained in a high yield by reacting a novel 8,2'-anhydro-8-mercapto-9-arabinofuranosyl purine derivative wherein the hydroxy group at the 5'-position is protected, with a fluorinating agent to achieve fluorination while retaining the steric configuration.

Furthermore, they have found that a desired purine nucleoside derivative which is fluorinated at the 3'-position (preferably the α-position) can be easily synthesized by removing a sulfur atom bonded at the 8-position and the 2'-position of the 8,2'-anhydro-3'-deoxy-3'-fluoro-8-mercapto-9-arabinofuranosyl purine derivative, deprotecting the 5'-position hydroxyl group, and, where necessary, substituent conversion on a nucleic acid base, and the like.

Accordingly, the present invention provides the following.
(i) A method of producing a fluorinated purine nucleoside represented by formula (2): 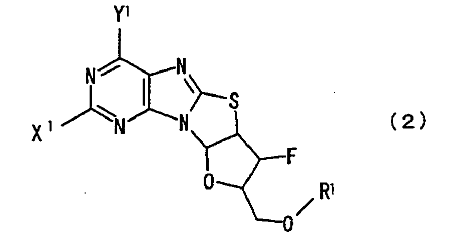 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group (hereinafter to be also referred to as Compound (2)), which comprises reacting a purine nucleoside represented by formula (1): 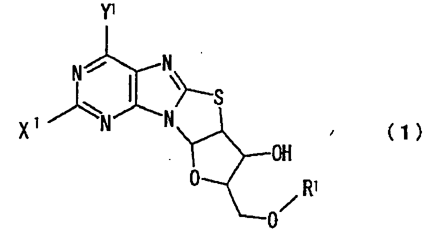 wherein R¹, X¹, and Y¹ are as defined above (hereinafter to be also referred to as Compound (1)), with a fluorinating agent.
(2) A method of producing a fluorinated purine nucleoside represented by formula (3): 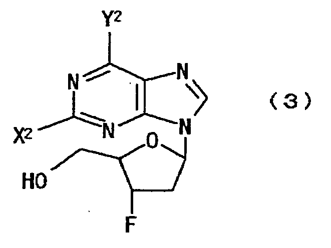 wherein X² is a hydrogen atom, a halogen atom, an optionally substituted amino group, or an optionally substituted hydroxyl group; and Y² is a hydrogen atom, a halogen atom, an optionally substituted amino group, or an optionally substituted hydroxyl group (hereinafter to be also referred to as Compound (3)), which comprises subjecting a fluorinated purine nucleoside represented by formula (2): 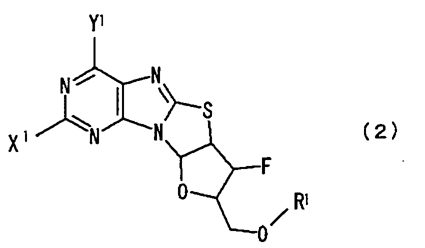 wherein R¹ is a hydroxyl-protecting group, X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, to, in any order:
   (A) desulfurizing to remove sulfur atom,
   (B) deprotecting to eliminate R¹, and
   (C) when X¹ and X² are different groups, and/or Y¹ and Y² are different groups, a converting X¹ to X² and/or Y¹ to Y².
(3) A method of producing a fluorinated purine nucleoside represented by formula (3): 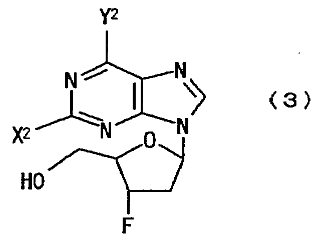 wherein X² is a hydrogen atom, a halogen atom, an optionally substituted amino group, or an optionally substituted hydroxyl group; and Y² is a hydrogen atom, a halogen atom, an optionally substituted amino group, or an optionally substituted hydroxyl group, which comprises reacting a purine nucleoside represented by formula (1): 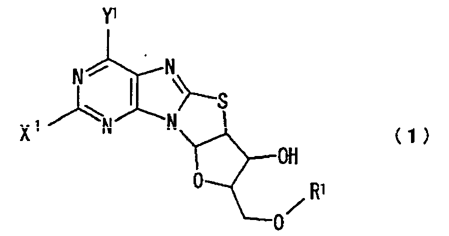 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, with a fluorinating agent to obtain a fluorinated purine nucleoside represented by formula (2): 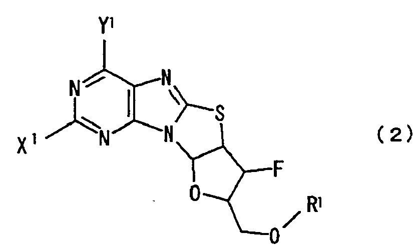 wherein R¹, X¹, and Y¹ are as defined above, and then subjecting the fluorinated purine nucleoside to, in any order:
   (A) desulfurizing to remove sulfur atom,
   (B) deprotecting to eliminate R¹, and
   (C) when X¹ and X² are different groups, and/or Y¹ and Y² are different groups, converting X¹ to X² and/or Y¹ to Y².
(4) A method of producing a purine nucleoside represented by formula (1): 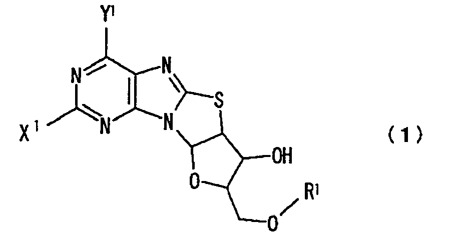 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, which comprises subjecting a purine nucleoside represented by formula (A): 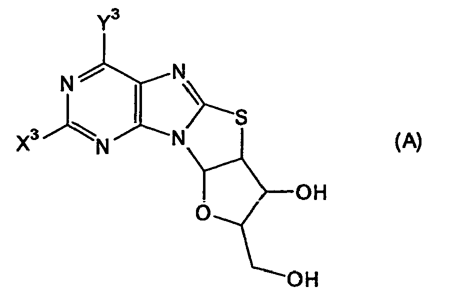 wherein X³ is a hydrogen atom, an amino group, or a hydroxyl group; and Y³ is a hydrogen atom, an amino group, or a hydroxyl group. (hereinafter to be also referred to as Compound (A)), to, in any order:
   (D) introducing R¹ into the 5'-position hydroxyl group to protect the hydroxyl group, and
   (E) when X³ and X¹ are different groups, and/or when Y³ and Y¹ are different groups, converting X³ to X¹ and/or Y³ to Y¹.
(5) A method of producing a fluorinated purine nucleoside represented by formula (3): 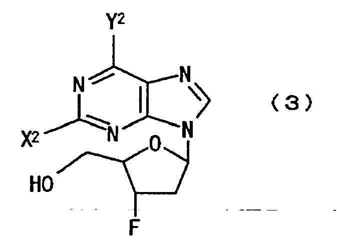 wherein X² is a hydrogen atom, a halogen atom, an optionally substituted amino group, or an optionally substituted hydroxyl group; and Y² is a hydrogen atom, a halogen atom, an optionally substituted amino group, or an optionally substituted hydroxyl group, which comprises subjecting a purine nucleoside represented by formula (A): 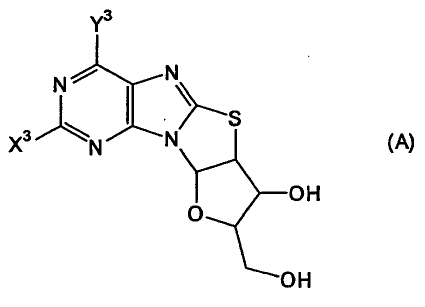 wherein X³ is a hydrogen atom, an amino group or a hydroxyl group; and Y³ is a hydrogen atom, an amino group or a hydroxyl group, to, in any order:
   (D) introducing R¹ into the 5'-position hydroxyl group to protect the hydroxyl group, and
   (E) when X³ and X¹ are different groups, and/or when Y³ and Y¹ are different groups, converting X³ to X¹ and/or Y³ to Y¹, to obtain a purine nucleoside represented by formula (1): 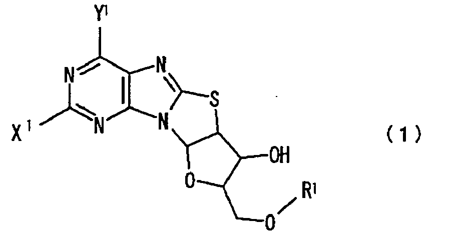 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, reacting the purine nucleoside with a fluorinating agent to obtain a fluorinated purine nucleoside represented by formula (2): 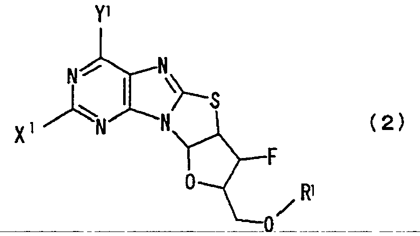 wherein R¹, X¹ and Y¹ are as defined above, and then subjecting the fluorinated purine nucleoside to, in any order:
      (A) desulfurizing to remove sulfur atom,
      (B) deprotecting step to eliminate R¹, and
      (C) when X¹ and X² are different groups, and/or Y¹ and Y² are different groups, converting X¹ to X² and/or Y¹ to Y².
(6) The method of any one of the above-mentioned (1), (3) and (5), wherein the fluorinating agent is perfluoroalkanesulfonyl fluoride and the reacting with a fluorinating agent is carried out in the presence of a base.
(7) The method of any one of the above-mentioned (1) and (3) to (5), wherein the purine nucleoside represented by formula (1) has a relative configuration represented by formula (1'): 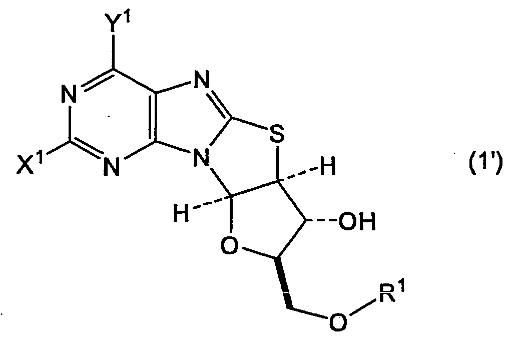 wherein R¹, X¹, and Y¹ are as defined in the above-mentioned (1).
(8) The method of any one of the above-mentioned (1) to (3) and (5), wherein the fluorinated purine nucleoside represented by formula (2) has a relative configuration represented by formula (2'): 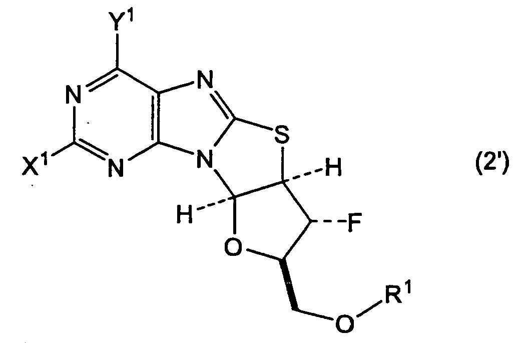 wherein R¹, X¹, and Y¹ are as defined in the above-mentioned (1).
(9) The method of any one of the above-mentioned (2), (3) and (5), wherein the fluorinated purine nucleoside represented by formula (3) has a relative configuration represented by formula (3'): 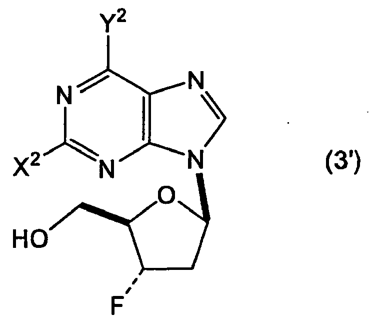 wherein X² and Y² are as defined in the above-mentioned (2).
(10) The method of the above-mentioned (4) or (5), wherein the purine nucleoside represented by formula (A) has a relative configuration represented by formula (A'): 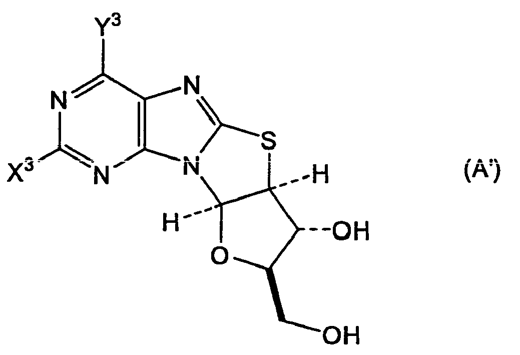 wherein X³ and Y³ are as defined in the above-mentioned (4).
(11) A method of producing a fluorinated purine nucleoside represented by formula (5): 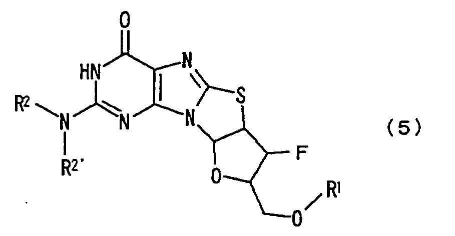 wherein R¹ is a hydroxyl-protecting group; and R² and R^{2'} are each independently a hydrogen atom or an amino-protecting group, or R² and R^{2'} in combination optionally form an amino-protecting group (hereinafter to be also referred to as Compound (5)), which comprises reacting a purine nucleoside represented by formula (4): 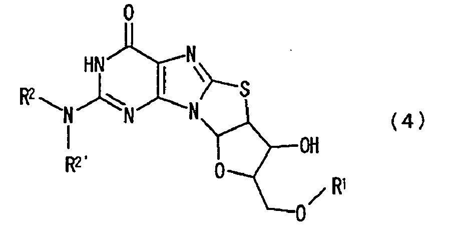 wherein R¹, R², and R^{2'} are as defined above (hereinafter to be also referred to as Compound (4)), with a fluorinating agent.
(12) A method of producing a fluorinated purine nucleoside represented by formula (6): 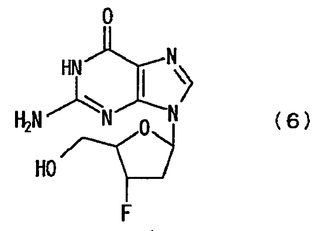 (hereinafter to be also referred to as Compound (6)), which comprises subjecting a fluorinated purine nucleoside represented by formula (5): 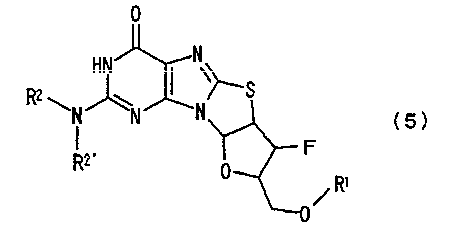 wherein R¹ is a hydroxyl-protecting group; and R² and R^{2'} are each independently a hydrogen atom or an amino-protecting group, or R² and R^{2'} in combination optionally form an amino-protecting group, to, in any order:
   (A) desulfurizing to remove sulfur atom,
   (B) deprotecting step to eliminate R¹, and
   (C') when R² and/or R^{2'} are/is amino-protecting group(s), deprotecting to eliminate the protecting group.
(13) A method of producing a fluorinated purine nucleoside represented by formula (6): 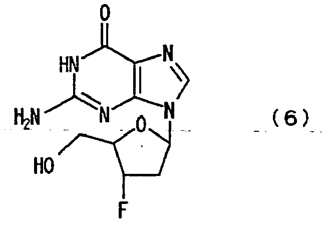 which comprises reacting a purine nucleoside represented by formula (4): 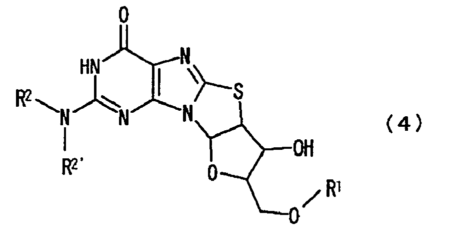 wherein R¹ is a hydroxyl-protecting group; and R² and R^{2'} are each independently a hydrogen atom or an amino-protecting group, or R² and R^{2'} in combination optionally form an amino-protecting group, with a fluorinating agent to obtain a fluorinated purine nucleoside represented by formula (5): 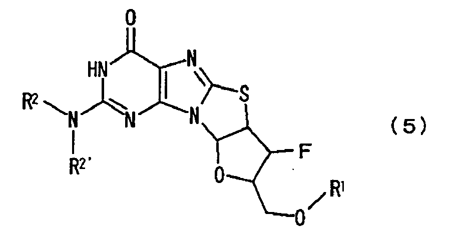 wherein R¹, R² and R^{2'} are as defined above, and subjecting the fluorinated purine nucleoside to, in any order:
   (A) desulfurizing to remove sulfur atom,
   (B) deprotecting to eliminate R¹, and
   (C') when R² and/or R^{2'} are/is amino-protecting group(s), deprotecting to eliminate the protecting group.
(14) A method of producing a purine nucleoside represented by formula (4): 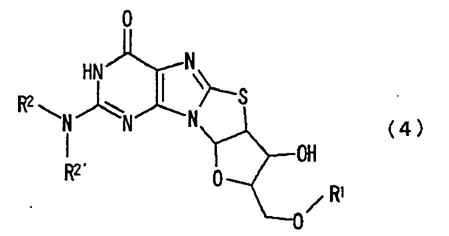 wherein R¹ is a hydroxyl-protecting group; and R² and R^{2'} are each independently a hydrogen atom or an amino-protecting group, or R² and R^{2'} in combination optionally form an amino-protecting group, which comprises subjecting a purine nucleoside represented by formula (B): 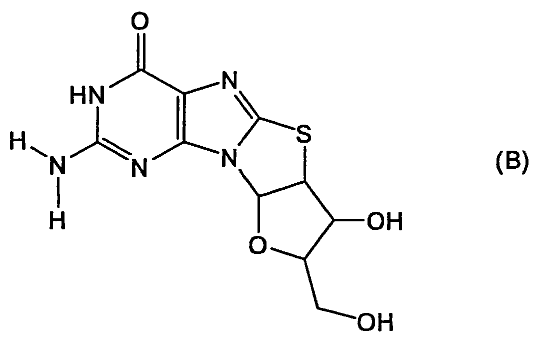 (hereinafter to be also referred to as Compound (B)), to, in any order:
   (D) introducing R¹ into the 5'-position hydroxyl group to protect the hydroxyl group, and
   (E') when R² and/or R^{2'} are/is amino-protecting group(s), introducing the protecting group.
(15) A method of producing a fluorinated purine nucleoside represented by formula (6): 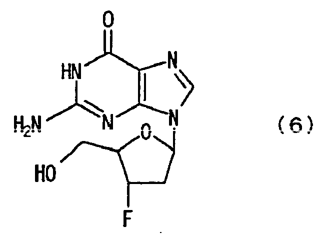 which comprises subjecting a purine nucleoside represented by formula (B): 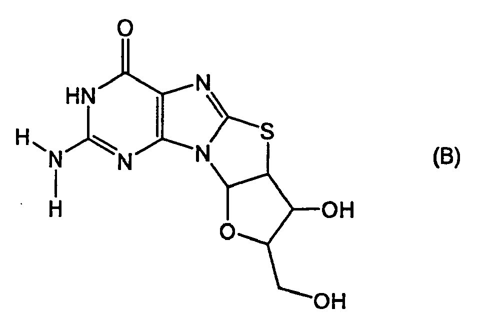 to, in any order:
   (D) introducing R¹ into the 5'-position hydroxyl group to protect the hydroxyl group, and
   (E') when R² and/or R^{2'} are/is amino-protecting group(s), introducing the protecting group, to obtain a purine nucleoside represented by formula (4): 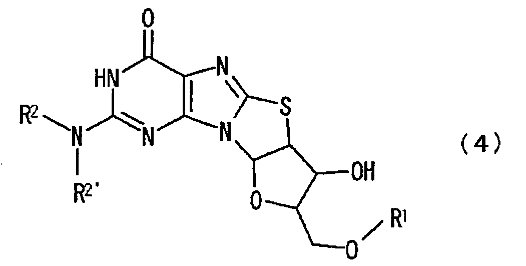 wherein R¹ is a hydroxyl-protecting group; and R² and R^{2'} are each independently a hydrogen atom or an amino-protecting group, or R² and R^{2'} in combination optionally form an amino-protecting group, reacting the purine nucleoside with a fluorinating agent to obtain a fluorinated purine nucleoside represented by formula (5): 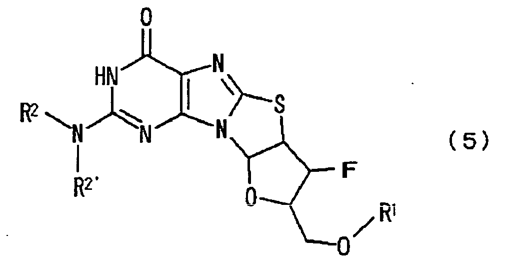 wherein R¹, R² and R^{2'} are as defined above, and then subjecting the fluorinated purine nucleoside to, in any order:
      (A) desulfurizing to remove sulfur atom,
      (B) deprotecting to eliminate R¹, and
      (C') when R² and/or R^{2'} are/is amino-protecting group(s), deprotecting to eliminate the protecting group.
(16) The method of any one of the above-mentioned (11), (13) and (15), wherein the fluorinating agent is perfluoroalkanesulfonyl fluoride and the reacting with a fluorinating agent is carried out in the presence of a base.
(17) The method of any one of the above-mentioned (11) and (13) to (15), wherein the purine nucleoside represented by formula (4) has a relative configuration represented by formula (4'): 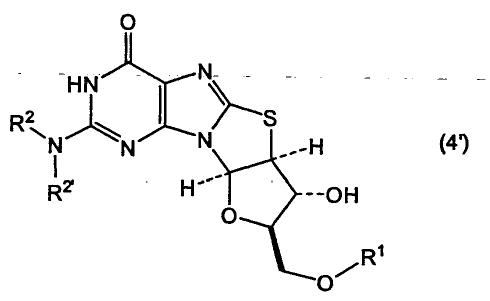 wherein R¹, R² and R^{2'} are as defined in the above-mentioned (11).
(18) The method of any one of the above-mentioned (11) to (13) and (15), wherein the fluorinated purine nucleoside represented by formula (5) has a relative configuration represented by formula (5'): 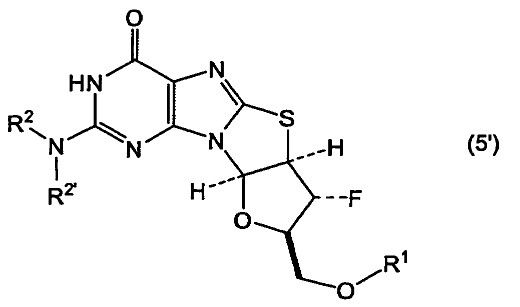 wherein R¹, R² and R^{2'} are as defined in the above-mentioned (11).
(19) The method of any one of the above-mentioned (12), (13) and (15), wherein the fluorinated purine nucleoside represented by formula (6) has a relative configuration represented by formula (6'): 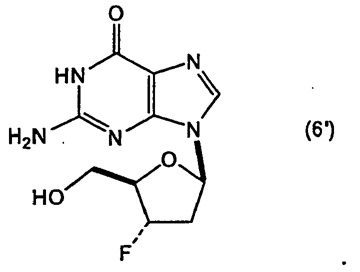
(20) The method of the above-mentioned (14) or (15), wherein the purine nucleoside represented by formula (B) has a relative configuration represented by formula (B'): 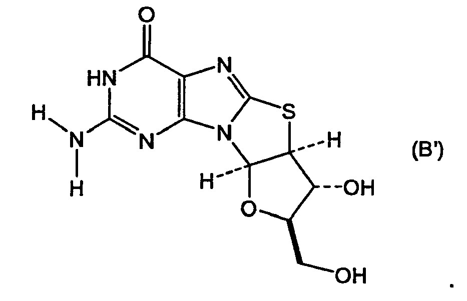
(21) The method of any one of the above-mentioned (11) to (15), wherein R¹ and R² are each a trityl group, a 9-phenylfluoren-9-yl group, or an acetyl group, each optionally having one or more substituents, and R^{2'} is a hydrogen atom.
(22) The method of any one of the above-mentioned (11) to (15), wherein R¹ is a trityl group optionally having one or more substituents, and R² and R^{2'} in combination form a dimethylaminomethylidene group.
(23) A 8,2'-anhydro-8-mercapto-9-arabinofuranosyl purine represented by formula (1): 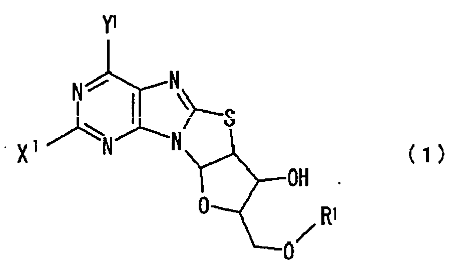 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group.
(24) The 8,2'-anhydro-8-mercapto-9-arabinofuranosyl purine of the above-mentioned (23), which has a relative configuration represented by formula (1'): 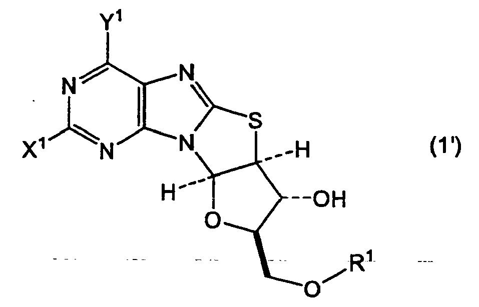 wherein R¹, X¹, and Y¹ are as defined in the above-mentioned (23).
(25) A 8,2'-anhydro-8-mercapto-9-arabinofuranosyl guanine represented by formula (4): 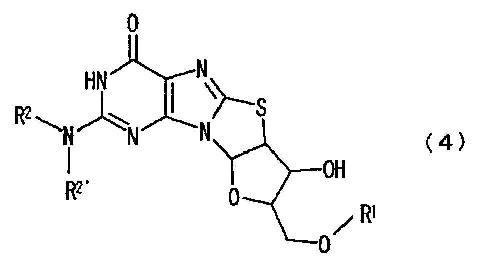 wherein R¹ is a hydroxyl-protecting group; and R² and R^{2'} are each independently a hydrogen atom or an amino-protecting group, or R² and R^{2'} in combination optionally form an amino-protecting group.
(26) The 8,2'-anhydro-8-mercapto-9-arabinofuranosyl guanine of the above-mentioned (25), which has a relative configuration represented by formula (4'): 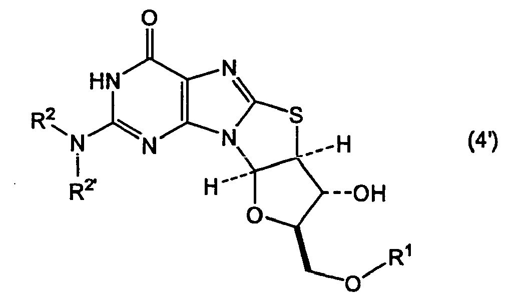 wherein R¹, R² and R²' are as defined in the above-mentioned (25).
(27) The 8,2'-anhydro-8-mercapto-9-arabinofuranosyl guanine of the above-mentioned (25) or (26), wherein R¹ and R² are each a trityl group, a 9-phenylfluoren-9-yl group or an acetyl group, each optionally having one or more substituents, and R^{2'} is a hydrogen atom.
(28) The 8,2'-anhydro-8-mercapto-9-arabinofuranosyl guanine of the above-mentioned (25) or (26), wherein R¹ is a trityl group optionally having one or more substituents, and R² and R^{2'} in combination form a dimethylaminomethylidene group.
(29) A 8,2'-anhydro-3'-deoxy-3'-fluoro-8-mercapto-9-arabinofuranosyl purine represented by formula (2): 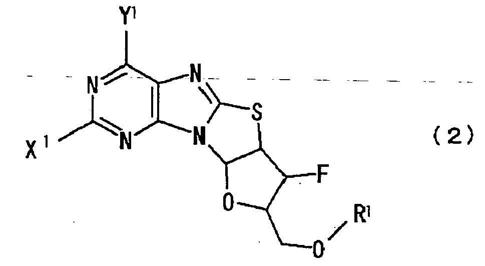 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group.
(30) The 8,2'-anhydro-3'-deoxy-3'-fluoro-8-mercapto-9-arabinofuranosyl purine of the above-mentioned (29), which has a relative configuration represented by formula (2'): 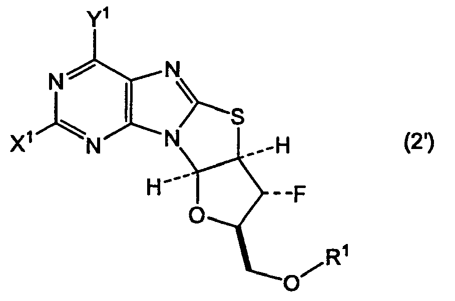 wherein R¹, X¹, and Y¹ are as defined in the above-mentioned (29).
(31) A 8,2'-anhydro-3'-deoxy-3'-fluoro-8-mercapto-9-arabinofuranosyl guanine represented by formula (5): 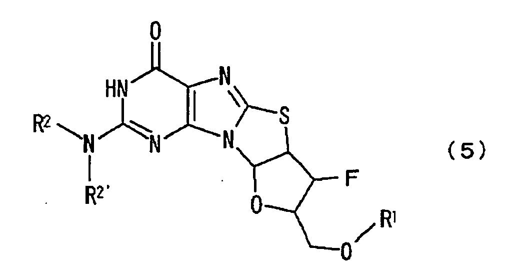 wherein R¹ is a hydroxyl-protecting group; and R² and R^{2'} are each independently a hydrogen atom or an amino-protecting group, or R² and R^{2'} in combination optionally form an amino-protecting group.
(32) The 8,2'-anhydro-3'-deoxy-3'-fluoro-8-mercapto-9-arabinofuranosyl guanine of the above-mentioned (31), which has a relative configuration represented by formula (5'): 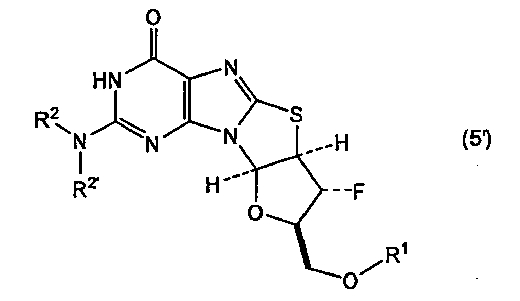 wherein R¹, R², and R²' are as defined in the above-mentioned (31).
(33) The 8,2'-anhydro-3'-deoxy-3'-fluoro-8-mercapto-9-arabinofuranosyl guanine of the above-mentioned (31) or (32), wherein R¹ and R² are each a trityl group, a 9-phenylfluoren-9-yl group or an acetyl group, each optionally having one or more substituents, and R^{2'} is a hydrogen atom.
(34) The 8,2'-anhydro-3'-deoxy-3'-fluoro-8-mercapto-9-arabinofuranosyl guanine of the above-mentioned (31) or (32), wherein R¹ is a trityl group optionally having one or more substituents, and R² and R^{2'} in combination form a dimethylaminomethylidene group.

The present invention provides an economic and efficient method of producing a purine nucleoside derivative which is fluorinated at the 3'-position (preferably the α-position), by fluorinating novel Compound (1) while retaining the steric configuration to obtain novel Compound (2) which is fluorinated at the 3'-position (preferably the α-position), and subjecting Compound (2) to desulfurization, deprotection, and the like to obtain desired Compound (3) which is fluorinated at the 3'-position (preferably the α-position) in a high yield, or similarly, fluorinating novel Compound (4) while retaining the steric configuration to obtain novel Compound (5) which is fluorinated at the 3'-position (preferably the α-position), and subjecting Compound (5) to desulfurization, deprotection, and the like to obtain desired Compound (6) which is fluorinated at the 3'-position (preferably the α-position) in a high yield, as well as intermediates therefor and a production method thereof, which are suitable as an industrial production method.

### DETAILED DESCRIPTION OF THE INVENTION

The present invention is described in detail in the following.

Respective symbols and terms used in the present specification are explained below.

In the formulas of the present invention, R¹ means a "hydroxyl-protecting group."
The "hydroxyl-protecting group" is not particularly limited as long as it does not adversely affect the reactions in the present invention, and can be easily eliminated after the reaction. As suitable protecting groups, for example, an acyl group, an alkyl group, an aralkyl group, a silyl group, and the like, each optionally having one or more substituents, can be mentioned.

As the "acyl group," for example, an acyl group having 1 to 7 carbon atoms such as a formyl group, an alkanoyl group having 2 to 7 carbon atoms (*e.g.*, acetyl group, pivaloyl group, *etc.),* a benzoyl group, and the like can be mentioned.

As the "alkyl group," for example, an alkyl group having 1 to 7 carbon atoms such as a methyl group, an ethyl group, an n-propyl group, an isopropyl group, an n-butyl group, an isobutyl group, a sec-butyl group, a tert-butyl group, a pentyl group, a hexyl group, a heptyl group, and the like can be mentioned.

As the "aralkyl group," for example, an aralkyl group having 7 to 22 carbon atoms such as a benzyl group, a trityl group, a 9-phenylfluoren-9-yl group, and the like can be mentioned.

As the "silyl group," for example, a silyl group trisubstituted with C1-6 alkyl groups (*e.g.*, methyl group, ethyl group, propyl group, isopropyl group, butyl group, isobutyl group, sec-butyl group, tert-butyl group, pentyl group, hexyl group, *etc*.), such as a trimethylsilyl group, a triethylsilyl group, a tert-butyldimethylsilyl group, and the like, can be mentioned.

As the substituent that the above-mentioned acyl group, alkyl group, aralkyl group, silyl group, and the like may each have, for example, a halogen atom (*e.g.*, fluorine atom, chlorine atom, bromine atom, iodine atom), a C1-6 alkyl group (*e.g.*, methyl group, ethyl group, propyl group, isopropyl group, butyl group, isobutyl group, sec-butyl group, tert-butyl group, pentyl group, hexyl group, *etc.),* a C6-14 aryl group (*e.g.*, phenyl group, naphthyl group, anthryl group, *etc.),* a C1-6 alkoxy group (*e.g.,* methoxy group, ethoxy group, propoxy group, isopropoxy group, butoxy group, isobutoxy group, sec-butoxy group, tert-butoxy group, pentyloxy group, hexyloxy group, *etc.),* a C1-6 alkylthio group (*e.g.,* methylthio group, ethylthio group, propylthio group, isopropylthio group, butylthio group, isobutylthio group, sec-butylthio group, tert-butylthio group, pentylthio group, hexylthio group, *etc.),* a mono- or di-(C1-6 alkyl)amino group (C1-6 alkyl moiety is exemplified by methyl group, ethyl group, propyl group, isopropyl group, butyl group, isobutyl group, sec-butyl group, tert-butyl group, pentyl group, hexyl group, and the like), and the like can be mentioned.

While the number of substituents and the position thereof are not particularly limited, the number of substituents is preferably 1 to 10, more preferably 1 to 3.

In addition to the above-mentioned hydroxyl-protecting groups, hydroxyl-protecting groups known to those of ordinary skill in the art, such as a methoxymethyl group, a methylthiomethyl group, a benzyloxymethyl group, a methoxyethoxymethyl group, a tetrahydropyranyl group, a methoxycarbonyl group, a 9-fluorenylmethoxycarbonyl group, a 2,2,2-trichloroethoxycarbonyl group, a benzyloxycarbonyl group, a tert-butoxycarbonyl group, and the like, may be used. Of the above-mentioned hydroxyl-protecting groups, preferred are an acetyl group, a benzoyl group, a trityl group, a 4-monomethoxytrityl group, a 4,4'-dimethoxytrityl group, and a 9-phenylfluoren-9-yl group, and particularly preferred are a trityl group, a 9-phenylfluoren-9-yl group, a 4-monomethoxytrityl group, and a 4,4'-dimethoxytrityl group.

In the formulas of the present invention, R² and R^{2'} are each independently a hydrogen atom or an amino-protecting group.

The "amino-protecting group" is not particularly limited as long as it does not adversely affect the reactions in the present invention. For example, an aralkyl group, an acyl group, an alkoxycarbonyl group, and the like, each optionally having one or more substituents, can be mentioned.

As the "aralkyl group," for example, an aralkyl group having 7 to 22 carbon atoms such as a benzyl group, a trityl group, a 9-phenylfluoren-9-yl group, and the like can be mentioned.

As the "acyl group," for example, an acyl group having 1 to 10 carbon atoms such as a formyl group, an alkanoyl group having 2 to 10 carbon atoms (*e.g.*, acetyl group, pivaloyl group, *etc.),* a benzoyl group, a phenylacetyl group, and the like can be mentioned.

As the "alkoxycarbonyl group," for example, an alkoxycarbonyl group having 2 to 8 carbon atoms such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, a hexyloxycarbonyl group, a benzyloxycarbonyl group, and the like can be mentioned.

R² and R^{2'} in combination may form an amino-protecting group. As the "amino-protecting group" formed in this manner, an alkylidene group, a group that forms an imido group together with a nitrogen atom of an amino group, and the like can be mentioned.

As the "alkylidene group," for example, an alkylidene group having 1 to 8 carbon atoms and optionally having one or more substituents, such as a benzylidene group, a diphenylmethylidene group, a bis(methylthio)methylidene group, a dimethylaminomethylidene group, and the like, can be mentioned.

As the "imido group" formed together with a nitrogen atom of an amino group, a phthalimido group, a succinimido group, a maleimido group, and the like can be mentioned.

As the substituent that the above-mentioned aralkyl group, acyl group, alkoxycarbonyl group, alkylidene group, and the like may each have, for example, those similar to the substituents that the "acyl group" of the "acyl group optionally having one or more substituents" for the "hydroxyl-protecting group" represented by R¹ may have can be mentioned.

While the number of substituents and the position thereof are not particularly limited, the number of substituents is preferably 1 to 6, more preferably 1 to 3. Of the above-mentioned amino-protecting groups, preferred are an acetyl group, a benzoyl group, a trityl group, a 4-monomethoxytrityl group, a 4,4'-dimethoxytrityl group, and a 9-phenylfluoren-9-yl group, and particularly preferred are a trityl group, a 9-phenylfluoren-9-yl group, a 4-monomethoxytrityl group, and a 4,4'-dimethoxytrityl group.

When R² and R²' in combination form an amino-protecting group, the amino-protecting group is preferably an alkylidene group optionally having one or more substituents, and particularly preferably a dimethylaminomethylidene group.

R² is preferably an amino-protecting group, and more preferably an acetyl group, a benzoyl group, a trityl group, a 4-monomethoxytrityl group, a 4,4'-dimethoxytrityl group, or a 9-phenylfluoren-9-yl group.

R^{2'} is particularly preferably a hydrogen atom.

In the formulas of the present invention, X¹ and Y¹ are each independently a hydrogen atom, a halogen atom, an optionally substituted amino group, or an optionally substituted hydroxyl group.

The substituent of the above-mentioned "optionally substituted amino group" and "optionally substituted hydroxyl group" includes a "protecting group" and a "modifying group."

In the present specification, the "protecting group" means a group intended to be eliminated after a desired conversion of another part of a compound, and the "modifying group" means a group introduced for the purpose of changing chemical property or physiological activity, which is not intended to be eliminated even after a desired change of another part of a compound is complete.

As the "halogen atom," a chlorine atom, a fluorine atom, a bromine atom, and an iodine atom can be mentioned.

The "protecting group" included in the substituent of the "optionally substituted amino group" is not particularly limited as long as it does not adversely affect the reactions in the present invention, and, for example, those similar to the groups exemplified for the "amino-protecting group" represented by R² or R^{2'} can be mentioned.

Of the above-mentioned protecting groups, preferred are an acetyl group, a benzoyl group, a trityl group, a 4-monomethoxytrityl group, a 4,4'-dimethoxytrityl group, a 9-phenylfluoren-9-yl group, and a dimethylaminomethylidene group, and particularly preferred are a trityl group, a 9-phenylfluoren-9-yl group, a 4-monomethoxytrityl group, and a 4,4'-dimethoxytrityl group.

The "modifying group" included in the substituent of the "optionally substituted amino group" is not particularly limited as long as it does not adversely affect the reactions in the present invention and, for example, an alkyl group and a haloalkyl group can be mentioned. In addition, the optionally substituted amino group may be a monosubstituted amino group or a disubstituted amino group.

As the "alkyl group," for example, an alkyl group having 1 to 10 carbon atoms such as a methyl group, an ethyl group, an n-propyl group, an isopropyl group, an n-butyl group, an isobutyl group, a sec-butyl group, a tert-butyl group, a cyclopropyl group, a pentyl group, a hexyl group, a heptyl group, an octyl group, a nonyl group, a decyl group, and the like can be mentioned.

As the "haloalkyl group," a haloalkyl group having 1 to 10 carbon atoms (*e.g.*, the above-mentioned alkyl group having 1 to 10 carbon atoms and 1 to 21 halogen atoms (fluorine atom, chlorine atom, bromine atom, iodine atom), such as trifluoromethyl group and the like) can be mentioned.

The "protecting group" included in the substituent of the "optionally substituted hydroxyl group" is not particularly limited as long as it does not adversely affect the reactions in the present invention and, for example, those similar to the groups exemplified for the "hydroxyl-protecting group" represented by R¹ can be mentioned.

The "modifying group" included in the substituent of the "optionally substituted hydroxyl group" is not particularly limited as long as it does not adversely affect the reactions in the present invention and, for example, those similar to the groups exemplified for the "modifying group" of the "optionally substituted amino group" represented by X¹ or Y¹ can be mentioned.

In the formulas of the present invention, X² and Y² are each independently a hydrogen atom, a halogen atom, an optionally substituted amino group, or an optionally substituted hydroxyl group, which is defined in the same manner as for the above-mentioned X¹ or Y¹.

In the formulas of the present invention, X³ and Y³ are each independently a hydrogen atom, a halogen atom, an optionally substituted amino group, or an optionally substituted hydroxyl group, which is defined in the same manner as for the above-mentioned X¹ or Y¹.

Various purine base moieties of purine nucleoside can be constituted by combining the above-mentioned specific examples of X¹ and Y¹ (or X² and Y²) and each combination of X¹ and Y¹ (or X² and Y²) is sometimes known by a specific name. The following Tables exemplarily show combinations of X¹ and Y¹ (or X² and Y²), and the name of the purine base corresponding to the combination. Those of ordinary skill in the art will appreciate that the present invention is not limited to the following.

**Table 1-1**

| | X¹(X²) | Y¹(Y²) | Name |
|---|---|---|---|
| 1 | H | H | purine |
| 2 | H | NH₂ | adenine |
| 3 | NH₂ | OH | guanine |
| 4 | H | OH | hypoxanthine |
| 5 | OH | OH | xanthine |
| 6 | NH₂ | H | 2-aminopurine |
| 7 | AcNH | H | 2-acetylaminopurine |
| 8 | H | F | 6-fluoropurine |
| 9 | H | Cl | 6-chloropurine |
| 10 | H | MeNH | 6-methylaminopurine |
| 11 | H | Me₂N | 6-dimethylaminopurine |
| 12 | H | CF₃NH | 6-trifluoromethylaminopurine |
| 13 | H | ^{c}PrNH | 6-cyclopropylaminopurine |
| 14 | H | AcNH | 6-acetylaminopurine |
| 15 | H | BzNH | 6-benzoylaminopurine |
| 16 | F | NH₂ | 2-fluoro-6-aminopurine |
| 17 | Cl | NH₂ | 2-chloro-6-aminopurine |
| 18 | Cl | Cl | 2,6-dichloropurine |
| 19 | H | MeO | 6-methoxypurine |
| 20 | H | BnO | 6-benzyloxypurine |

**Table 1-2**

| | | | |
|---|---|---|---|
| 21 | H | AcO | 6-acetoxypurine |
| 22 | H | BzO | 6-benzoyloxypurine |
| 23 | NH₂ | NH₂ | 2,6-diaminopurine |
| 24 | NH₂ | ^{c}PrNH | 2-amino-6-cyclopropylaminopurine |
| 25 | NH₂ | Cl | 2-amino-6-chloropurine |
| 26 | AcNH | Cl | 2-acetylamino-6-chloropurine |
| 27 | NH₂ | BnO | 2-amino-6-benzylozypurine |
| 28 | AcNH | BnO | 2-acetylamino-6-benzyloxypurine |
| 29 | AcNH | OH | N-acetylguanine |
| 30 | PhCH₂CONH | OH | N-(phenylacetyl)guanine |
| 31 | Ph₃CNH | OH | N-tritylguanine |
| 32 | (p-MeOC₆H₄)Ph₂CNH | OH | N-(4-monomethoxytrityl)guanine |
| 33 | (p-MeOC₆H₄)₂PhCNH | OH | N-(4,4'-dimethoxytrityl)guanine |
| 34 | (9-phenylfluoren-9-yl)amino | OH | N-(9-phenylfluoren-9-yl)guanine |
| 35 | Me₂NC=N | OH | N-(dimethylaminomethylidene)guanine |

Of the combinations of X¹ and Y¹, preferred are (X¹=H, Y¹=NH₂), (X¹=NH₂, Y¹=OH), (X¹=H, Y¹=OH), (X¹=AcNH, Y¹=OH), (X¹=Ph₃CNH, Y¹=OH), (X¹=(P-MeOC₆H₄)Ph₂CNH, Y¹=OH), (X¹=(p-MeOC₆H₄)₂PhCNH, Y¹=OH), (X¹=Me₂NC=N, Y¹=OH), and the like, and particularly preferred are (X¹=Ph₃CNH, Y¹=OH), (X¹=(p-MeOC₆H₄)Ph₂CNH, Y¹=OH), and (X¹=(p-MeOC₆H₄)₂PhCNH, Y¹=OH).

Of the combinations of X² and Y², preferred are (X²=H, Y²=NH₂) (adenine), (X²=NH₂, Y²=OH) (guanine), and (X²=H, Y²=OH) (hypoxanthine), and particularly preferred is (X²=NH₂, Y²=OH) (guanine).

The method of producing the purine nucleoside and the method of producing an intermediate thereof of the present invention are explained in the following.

### Production method of Compound (1).

Novel Compound (1) can be produced by subjecting Compound (A) to the following Steps (D) and (E) in any order.
(D) introducing R¹ into the 5'-position hydroxyl group to protect the hydroxyl group, and
(E) when X³ and X¹ are different groups, and/or when Y³ and Y¹ are different groups, converting X³ to X¹ and/or Y³ to Y¹.

The starting material, Compound (A), is a known compound and can be produced according to the method described, for example, in Chem. Pharm. Bull., vol. 15, p. 94 (1967) (X³=H, Y³=NH₂); Chem. Pharm. Bull., vol. 20, p. 635 (1972) (X³=H, Y³=H) and (X³=OH, Y³=OH); Chem. Pharm. Bull., vol. 29, p. 3449 (1981) (X³ =NH₂, Y³=NH₂); Can. J. Chem., vol. 50, p. 1100 (1972) (X³ =NH₂, Y³=OH), and the like.

In Step (D), a hydroxyl-protecting group defined for R¹ is introduced into the 5'-position hydroxyl group of Compound (A) for protection. Conventionally known methods *(see, e.g.,* Protecting Groups in Organic Synthesis 3rd edition, John Wiley & Sons, Inc. 1999) can be employed depending on the kind of the protecting group.

In the following, a preferable embodiment wherein the protecting group is an aralkyl group (*e.g.*, trityl group, 9-phenylfluoren-9-yl group etc.) is explained, which is not to be construed as limiting Step (D).

Step (D) for introducing an aralkyl group as a protecting group can be performed by reacting Compound (A) with aralkyl halide (*e.g.*, tritylchloride, 4,4'-dimethoxytritylchloride, 9-bromo-9-phenylfluorene, *etc.)* in the presence of a base.

As the base, triethylamine, pyridine, 4-dimethylaminopyridine, tributylamine, N,N-diisopropylethylamine, 1,8-diazabicyclo[5,4,0]undec-7-ene, and the like can be mentioned, and the amount of use thereof is generally 1 to 100 equivalents, preferably 1 to 3 equivalents, relative to Compound (A).

The amount of aralkyl halide to be used is 1 to 10 equivalents, preferably 1 to 3 equivalents, relative to Compound (A).

The protection reaction is generally carried out in a solvent, and the solvent is not particularly limited as long as it does not inhibit the reaction. For example, a single or mixed solvent of N,N-dimethylformamide, acetonitrile, pyridine, N,N-dimethylacetamide, N-methylpyrrolidone, and the like can be mentioned, with preference given to N,N-dimethylformamide and acetonitrile. The amount of the solvent to be used is generally 10- to 100-fold by weight relative to Compound (A).

The reaction temperature is generally within the range of 10°C to 60°C and the reaction time is generally 1 to 24 hours.

Step (E) is appropriately performed as necessary when X³ and X¹ are different groups, and/or when Y³ and Y¹ are different groups, which specifically comprises protection, deprotection and/or modification to convert X³ to X¹ and/or Y³ to Y¹.

The "protection" in Step (E) means, for example, when X³ and/or Y³ are/is an amino group and/or a hydroxyl group, a step of introducing the above-mentioned protecting group and, the "deprotection" in Step (E) means elimination of the protecting group, both performed according to methods known *per se* (*see e.g.,* Protecting Groups in Organic Synthesis 3rd edition, John Wiley & Sons, Inc. 1999).

The "modification" in Step (E) means changing substituents, which is different from the above-mentioned "protection" and "deprotection." For example, when X³ and/or Y³ are/is an amino group and/or a hydroxyl group, it means a step of introducing the modifying group exemplified above or a step of converting to a halogen atom, a hydrogen atom and the like, and the like, which can be performed by a conventionally known method in the nucleic acid synthesis (*see e.g.,* "Nucleic acid Chemistry Part II", Leroy B. Townsend, R. Stuart Tipson, A Wiley-Interscience Publication).

Here, when X³ and/or Y³ of Compound (A) are/is an amino group or a hydroxyl group, a protecting group may be introduced by performing Step (E) before performing the above-mentioned Step (D). Alternatively, protection of Step (E) may be performed simultaneously with the protection of the hydroxyl group at the 5'-position in the above-mentioned Step (D). In this case, the amount of the protecting reagent and the like to be used may be determined according to the number of the groups to be protected. For example, in the case of X³=NH₂, both the hydroxyl group at the 5'-position and the amino group for X³ can be simultaneously protected with trityl group by reacting Compound (A) with trityl chloride under the basic conditions generally used (*e.g.*, triethylamine and the like).

When a protecting group is simultaneously introduced into a protectable group (*e.g.,* 3'-position hydroxyl group) present in Compound (A) by Steps (D) and (E), Compound (1) can also be produced by selectively deprotecting at the 3'-position. For example, when X³=NH₂, it is possible to deprotect only the 3'-position secondary hydroxyl group after protecting all of the 5'-position hydroxyl group, the amino group for X³, and the 3'-position hydroxyl group (*e.g.*, protecting with acyl group such as acetyl group *etc.)* in Compound (A).

The reaction to obtain a compound represented by formula (1) is a reaction to introduce a protecting group, and such protection reaction can be carried out by a conventionally known method. The details of the protection can be known from, for example, Protecting Groups in Organic Synthesis 3rd edition, John Wiley & Sons, Inc. 1999 and a detailed explanation is omitted here. For example, for protection with a trityl group, N,N-dimethylformamide is used as a solvent, triethylamine is added as a base, and trityl chloride and a compound represented by Compound (A) are reacted for a given time to afford a tritylated compound.

### Production method of Compound (2).

Novel Compound (2) can be obtained by reacting novel Compound (1) with a fluorinating agent.

As the "fluorinating agent" to be used for fluorination, a nucleophilic fluorinating agent can be used. For example, metal fluoride (potassium fluoride, cesium fluoride, etc.), ammonium fluoride (tetrabutylammonium fluoride etc.), sulfur fluorinating agent (dialkylaminosulfur trifluoride such as diethylaminosulfur trifluoride, morpholinosulfur trifluoride and the like, and the like), fluoroalkylamine fluorinating agent (diethylamine-hexafluoroheptene adduct, 2,2-difluoro-1,3-dimethylimidazolidinone etc.), perfluoroalkanesulfonyl fluoride (perfluorobutanesulfonyl fluoride, perfluorononanesulfonyl fluoride, etc.) and the like can be mentioned. Of these, perfluoroalkanesulfonyl fluoride and dialkylaminosulfur trifluoride are preferable. In view of possible scaling up, a perfluoroalkanesulfonyl fluoride is particularly preferable. Here, as the "alkane" moiety of the perfluoroalkanesulfonyl fluoride, an alkane having 1 to 10 carbon atoms such as butane, nonane and the like can be mentioned.

As regards the "alkyl" moiety of dialkylaminosulfur trifluoride, "alkyl group optionally having one or more substituents" exemplified for the "hydroxyl-protecting group" represented by R¹, such as ethyl, 2-methoxyethyl, and the like can be mentioned. The "dialkyl" may be integrated to form a ring, and as such example, morpholinosulfur trifluoride wherein dialkylamino is morpholino, and the like can be mentioned.

The amount of the fluorinating agent to be used is generally 1 to 30 equivalents, preferably 1 to 15 equivalents, relative to Compound (1). The above-mentioned various fluorinating agents may require other conditions such as basic conditions and the like and, for example, in the case of perfluoroalkanesulfonyl fluoride, fluorination needs to be carried out in the presence of a base. As the "base" to be used for providing basic conditions, for example, N,N-diisopropylethylamine, triethylamine, tributylamine, and the like can be mentioned, with preference given to N,N-diisopropylethylamine. The amount of the amine to be used is generally 1 to 30 equivalents, preferably 1 to 15 equivalents, relative to Compound (1).

This reaction is carried out in a reaction solvent. Preferable reaction solvent is not particularly limited as long as it does not adversely affect the reaction and, for example, ethyl acetate, isopropyl acetate, tetrahydrofuran, acetonitrile, dichloromethane, and the like can be mentioned. Of these, preferred are ethyl acetate and tetrahydrofuran. The amount of the solvent to be used is generally 5- to 50-fold weight, preferably 10- to 30-fold weight, relative to Compound (1). While the reaction time varies depending on the fluorinating agent to be used, it is generally 1 to 48 hours. When, for example, perfluoroalkanesulfonyl fluoride is used, it is 2 to 24 hours. While the reaction temperature also varies depending on the fluorinating agent to be used, it is generally, 20°C to 160°C and, for example, when perfluoroalkanesulfonyl fluoride is used, the reaction temperature is 40°C to 80°C.

The production method of the present invention is characterized by fluorination that proceeds while retaining the steric configuration.

### Production Method of Compound (3).

Compound (3) can be obtained by subjecting Compound (2) obtained above to each of the following steps in any order.
(A) desulfurizing to remove sulfur atom,
(B) deprotecting to eliminate R¹, and
(C) when X¹ and X² are different groups, and/or Y¹ and Y² are different groups, converting X¹ to X² and/or Y¹ to Y².

The conditions to be employed for Step (A) are not particularly limited as long as they do not cause a side reaction in the reaction site other than the object desulfurization reaction. A representative desulfurization method is a method using Raney-nickel.

When a desulfurization reaction is carried out by a method using Raney-nickel, examples of preferable solvent include 1-propanol, 1-butanol, and toluene. The amount of Raney-nickel to be used varies depending on the grade of activity thereof but is generally, 3- to 50-fold weight, preferably 5- to 20-fold weight, relative to Compound (2). In addition, a preferably reaction time for this reaction is 1 to 24 hours, and a preferable reaction temperature is 70°C to 150°C.

Step (B) is a step of eliminating the hydroxyl-protecting group R¹, and a deprotection method for each group recited as the aforementioned hydroxyl-protecting group is known (*e.g., see* Protecting,Groups in Organic Synthesis 3rd edition, John Wiley & Sons, Inc. 1999). For example, when R¹ is a trityl group, deprotection is carried out by stirring under conditions using a suitable amount of a suitable acid such as acetic acid, formic acid, trifluoroacetic acid, and the like at a suitable temperature (*e.g.*, 10°C to 100°C).

Step (C) is performed as necessary when X¹ and X² are different groups, and/or Y¹ and Y² are different groups. To be specific, it is a step of converting X¹ to X² and/or Y¹ to Y² by applying a protection, deprotection, and/or modification. Here, for the protection, an operation similar to the protection of the hydroxyl group or the amino group in the above-mentioned Step (E) can be performed. As for the protection, various protecting groups and protection reactions for respective groups are known, and as for the deprotection, various methods for the above-mentioned protecting groups are known. For example, Protecting Groups in Organic Synthesis 3rd edition, John Wiley & Sons, Inc. (1999) provides detailed explanation. In addition, the modification can be carried out in the same manner as in the above-mentioned Step (E).

The protection, deprotection, and/or modification in Step (C) may proceed simultaneously under the reaction conditions of Step (A) or Step (B). For example, when X¹ and/or Y¹ are/is an amino group substituted by a protecting group and/or a hydroxyl group substituted by a protecting group, the protecting group may be eliminated under the deprotection conditions of Step (B). In other words, the deprotection of Step (C) may proceed simultaneously with Step (B).

### Production Method of Compound (4).

Novel Compound (4) corresponds to the aforementioned Compound (1) wherein X¹=NR²R^{2'} (R² and R^{2'} are as defined above) and Y¹=OH and can be produced from Compound (B) by similar reactions as in the preparation of Compound (1). The detailed explanation of the production method of Compound (4) can be obtained from the production method of the aforementioned Compound (1), and therefore, is omitted here. Step (E') corresponds to the protection in Step (E).

### Production method of Compound (5).

Novel Compound (5) corresponding to the aforementioned Compound (2) wherein X¹=NR²R^{2'} (R² and R^{2'} are as defined above) and Y¹=OH (keto form) can be produced by similar reactions as in the preparation of Compound (2). The detailed explanation of the production method of Compound (5) can be obtained from the production method of the aforementioned Compound (2), and therefore, is omitted here.

### Production Method of Compound (6).

Compound (6) corresponds to the aforementioned Compound (3) wherein X²=NH₂ and Y²=OH (keto form) and can be produced by similar reactions as in the preparation of Compound (3). The detailed explanation of the production method of Compound (6) can be obtained from the production method of the aforementioned Compound (3), and therefore, is omitted here. Step (C') corresponds to the deprotection in Step (C).

The resultant products from each of the above-mentioned steps can be purified by isolation and purification methods generally known in chemical reactions. To be specific, various steps of chromatography, extraction, recrystallization, and the like can be used as appropriate, independently or in combination thereof.

In addition, those of ordinary skill in the art will appreciate that the steric configuration based on the asymmetric carbon atom in the compound used for the production method of the present invention is not particularly limited.

Examples of preferable structures of the compounds used for the production method of the present invention are shown in the following.

Compound (A) preferably has a relative configuration represented by the following formula (A'): 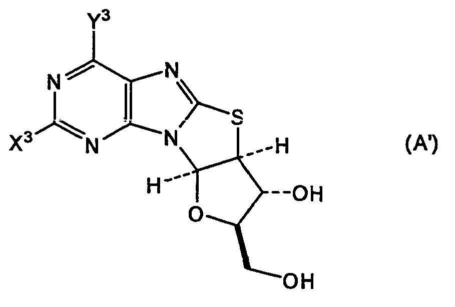 wherein each symbol is as defined above.

Particularly preferably, Compound (A) has the absolute configuration represented by the following formula (A"): 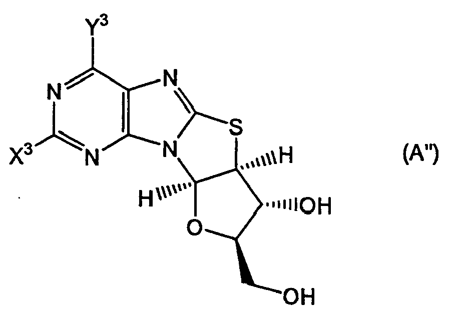 wherein each symbol is as defined above.

Compound (1) preferably has a relative configuration represented by the following formula (1'): 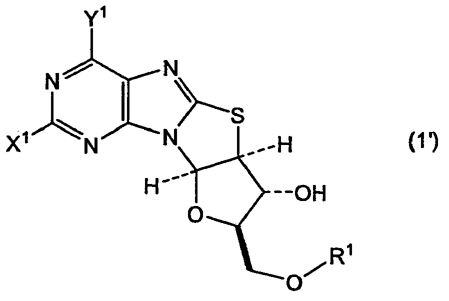 wherein each symbol is as defined above.

Particularly preferably, Compound (1) has the absolute configuration represented by the following formula (1"): 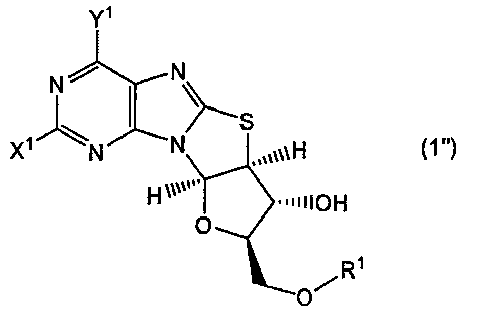 wherein each symbol is as defined above.

Compound (2) preferably has a relative configuration represented by the following formula (2'): 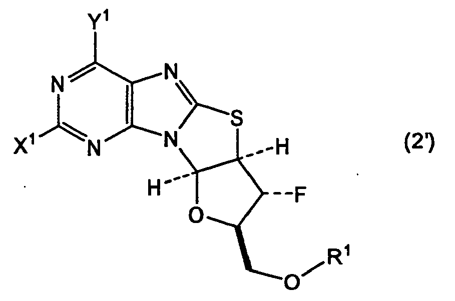 wherein each symbol is as defined above.

Particularly preferably, Compound (2) has the absolute configuration represented by the following formula (2"): 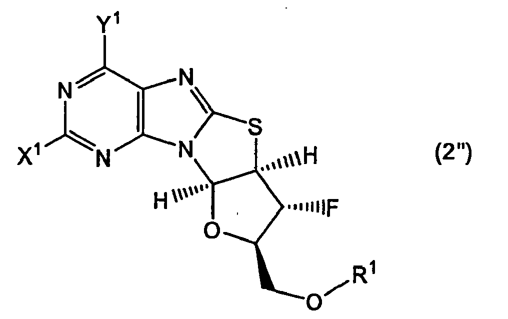 wherein each symbol is as defined above.

Compound (3) preferably has a relative configuration represented by the following formula (3'): 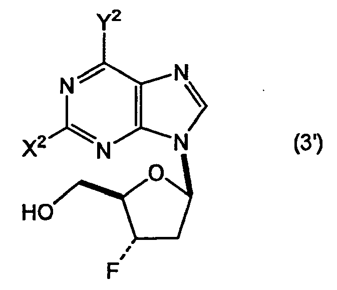 wherein each symbol is as defined above.

Particularly preferably, Compound (3) has the absolute configuration represented by the following formula (3"): 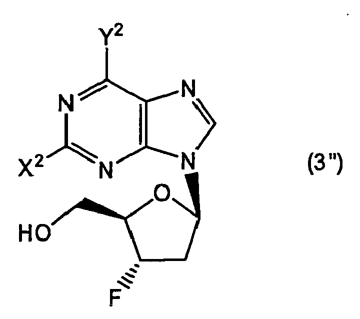 wherein each symbol is as defined above.

Compound (B) preferably has a relative configuration represented by the following formula (B'):

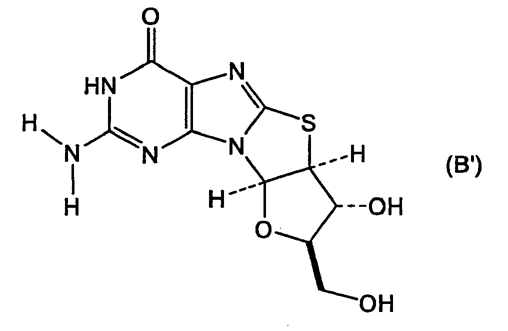

Particularly preferably, Compound (B) has the absolute configuration represented by the following formula (B"):

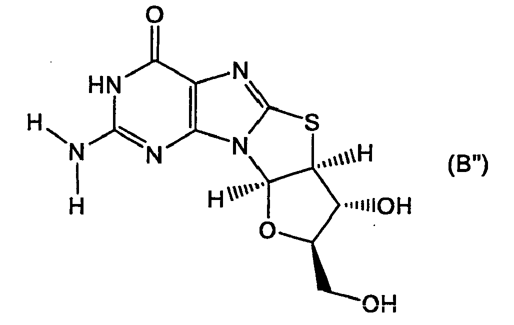

Compound (4) preferably has a relative configuration represented by the following formula (4'): 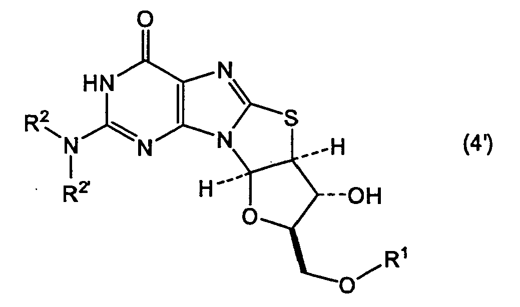 wherein each symbol is as defined above.

Particularly preferably, Compound (4) has the absolute configuration represented by the following formula (4"): 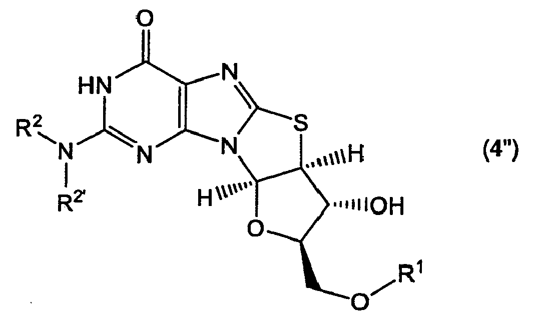 wherein each symbol is as defined above.

Compound (5) preferably has a relative configuration represented by the following formula (5'): 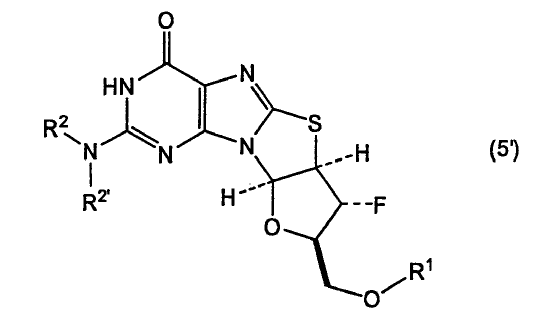 wherein each symbol is as defined above.

Particularly preferably, Compound (5) has the absolute configuration represented by the following formula (5"): 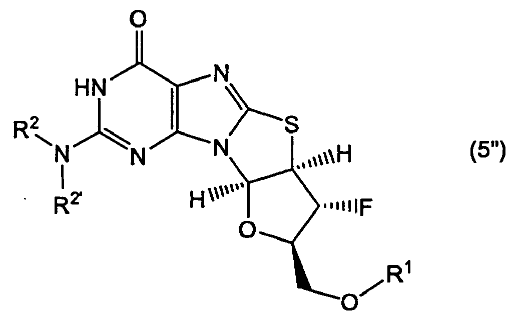 wherein each symbol is as defined above.

Compound (6) preferably has a relative configuration represented by the following formula (6'):

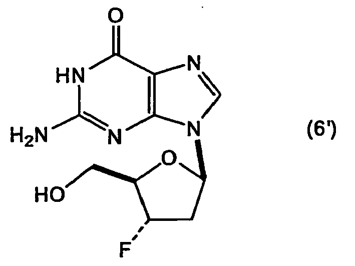

Particularly preferably, Compound (6) has the absolute configuration represented by the following formula (6"): 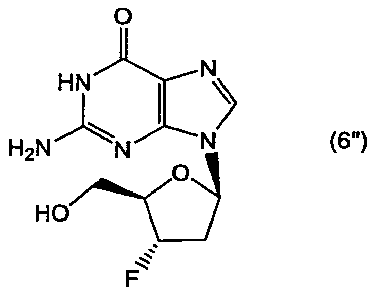 wherein each symbol is as defined above.

### Examples

Other features of the invention will become apparent in the course of the following descriptions of exemplary embodiments which are given for illustration of the invention and are not intended to be limiting thereof.

### Example 1. Synthesis of N²,5'-O-ditrityl-8,2'-anhydro-8-mercapto-9-β-arabinofuranosylguanine.

Triethylamine (7.03 mL, 50.4 mmol) and trityl chloride (14.6 g, 52.4 mmol) were added to an N,N-dimethylformamide (125 mL) solution of 8,2'-anhydro-8-mercapto-9-β-arabinofuranosylguanine (5 g, 16.8 mmol) synthesized according to the method described in Can. J. Chem., vol. 50, p. 1100 (1972), and the mixture was stirred with heating at 40°C for 20 hours. Methanol (50 mL) was added to the resulting solution to stop the reaction. The reaction solution was concentrated under reduced pressure. Dichloromethane (500 mL) and water (300 mL) were added to a concentrated residue to separate it into layers. Dichloromethane (300 mL) was added to the aqueous layer, and the mixture was re-extracted. The resulting two organic layers were mixed, and the mixture was dried over anhydrous magnesium sulfate and concentrated under reduced pressure. Toluene (73 mL) was added to the concentrated residue and the slurry was washed at room temperature for 12 hours. The thus-obtained solid was purified by chromatography (eluent: dichloromethane → dichloromethane:methanol=16:1) to obtain a desired product (7.88 g, yield of 60%) as a white solid.
¹H-NMR (CDCl₃)δ3.12 (dd, J=10.2, 5.6 Hz, 1H), 3.28 (dd, J=10.2, 5.2 Hz, 1H), 3.94 (br, 1H), 4.30-4.33 (m, 1H), 4.60-4.63 (m, 2H), 6.33 (d, J=5.9 Hz, 1H), 6.41 (br, 1H), 7.04-7.30 (m, 30H).
ESI MASS m/z 781.9 (M+H)⁺

### Example 2. Synthesis of N²,5'-O-ditrityl-8,2'-anhydro-3'-deoxy-3'-fluoro-8-mercapto-9-β-arabinofuranosylguanine.

Perfluorobutanesulfonyl fluoride (0.224 mL, 1.25 mmol) and N,N-diisopropylethylamine (0.218 mL, 1.25 mmol) were added to an ethyl acetate (2 mL) solution of N²,5'-O-ditrityl-8,2'-anhydro-8-mercapto-9-β-arabinofuranosylguanine (78.1 mg, 0.1 mmol), and the mixture was stirred with heating at 65°C for 20 hours. After the temperature was decreased to room temperature, ethyl acetate (5 mL) and water (2 mL) were added to the reaction solution to separate it into layers. The extract was dried over anhydrous magnesium sulfate and concentrated under reduced pressure. The concentrate was purified by chromatography (eluent: dichloromethane:methanol=32:1) to obtain a desired product (71.8 mg, yield of 91 %) as a white solid.
¹H-NMR (CDCl₃)δ3.03-3.09 (m, 1H), 3.18-3.23 (m, 1H), 4.60 (dt, J=22.9, 6.9 Hz, 1H), 4.83 (dd, J=20.4, 6.5 Hz, 1H), 5.29 (d, J=52.1 Hz, 1H), 6.34 (d, J=6.5 Hz, 1H), 6.43 (br, 1H), 7.05-7.40 (m, 30H).
ESI MASS m/z 784.4 (M+H)⁺

### Example 3. Synthesis of N²,5'-O-ditrityl-3'α-fluoro-2',3'-dideoxyguanosine.

1-Propanol (5 mL) was added to Raney nickel (550 mg, NDHT-90 manufactured by Kawaken Fine). The mixture was stirred at room temperature for 5 minutes and then allowed to stand still. The solvent was removed by decantation. This procedure was repeated once again, and toluene (5 mL) was then added. The mixture was stirred at room temperature for 5 minutes and then allowed to stand still. The solution was removed by decantation. Toluene (1 mL) and N²,5'-O-ditrityl-8,2'-anhydro-3'-deoxy-3'-fluoro-8-mercapto-9-β-arabinofuranosylguanine (39 mg, 0.05 mmol) were added, and the mixture was stirred with heating in an argon atmosphere at 90°C for 12 hours. After cooling, dichloromethane (15 mL) was added to the resulting reaction solution. Raney nickel was separated by filtration, and the filtrate was concentrated under reduced pressure. The concentrate was purified by chromatography (eluent: dichloromethane → dichloromethane:methanol = 16:1) to obtain a desired product (22.9 mg, yield of 61 %) as a white solid.
¹H-NMR (CDCl₃)δ2.00-2.30 (m, 2H), 3.20-3.30 (m, 2H), 4.25 (dm, J=26.6 Hz, 1H), 5.03 (dm, J=54.7 Hz, 1H), 5.57-5.62 (m, 1H), 7.15-7.45 (m, 31H).
ESI MASS m/z 754.3 (M+H)⁺

### Example 4. Synthesis of 3'α-fluoro-2,3'-dideoxyguanosine.

N²,5'-O-ditrityl-3'α-fluoro-2',3'-dideoxyguanosine (22.6 mg, 0.03 mmol) was added to a 80% acetic acid aqueous solution (0.3 mL), and the mixture was stirred with heating at 80°C for 2 hours. The resulting solution was concentrated under reduced pressure, and the thus-obtained solid was purified by chromatography (eluent: dichloromethane:methanol=4:1) to obtain a desired product (5.6 mg, yield of 69%) as a white solid.
¹H-NMR (DMSO-d₆)δ2.50-2.67 (m, 1H), 2.70-2.90 (m, 1H), 3.50-3.60 (m, 2H), 4.16 (dt, J=27.0, 4.9 Hz, 1H), 5.15 (t, J=5.4 Hz, 1H), 5.38 (dd, J=53.6, 4.3 Hz, 1H), 6.15 (dd, J=9.4, 5.6 Hz, 1H), 6.49 (br, 2H), 7.94 (s, 1H), 10.54 (br, 1H).
ESI MASS m/z 270.2 (M+H)⁺

### Example 5. Synthesis of N²,5'-O-diacetyl-8,2'-anhydro-8-mercapto-9-β-arabinofuranosylguanine.

A 28% sodium methoxide methanol solution (total 0.84 mL, 4.1 mmol) was added to a methanol solution (30 mL) of N²,3',5'-O-triacetyl-8,2'-anhydro-8-mercapto-9-β-arabinofuranosylguanine (1.52 g, 3.6 mmol), synthesized according to the method described in Chem. Pharm. Bull., vol. 29, p. 3449 (1981), in amounts of 0.3 mL, 0.3 mL, 0.12 mL, 0.06 mL, and 0.06 mL over 80 minutes at intervals of 20 minutes. Subsequently, a 6 mol/L hydrochloric acid aqueous solution (0.7 mL) was added to the reaction solution to stop the reaction, and the mixture was concentrated under reduced pressure. The concentrate was purified by chromatography (eluent: dichloromethane:methanol 16:1 → 4:1) to obtain a desired product (423 mg, yield of 31 %) as a white solid.
¹H-NMR (DMSO-d₆)δ1.91 (s, 3H), 2.16 (s, 3H), 3.95-4.00 (m, 1H), 4.10-4.18 (m, 2H), 4.33-4.38 (m, 1H), 4.84 (dd, J=6.7, 3.0 Hz, 1H), 6.09 (br, 1H), 6.47 (d, J=6.7 Hz, 1H). ESI MASS m/z 382.2 (M+H)⁺

### Example 6. Synthesis of N²,5'-O-diacetyl-8,2'-anhydro-3'-deoxy-3'-fluoro-8-mercapto-9-β-arabinofuranosylguanine.

Diethylaminosulfur trifluoride (0.99 mL, 1.0 mmol) was added to a dichloromethane (4 mL) suspension of N²,5'-O-diacetyl-8,2'-anhydro-8-mercapto-9-β-arabinofuranosylguanine (76.3 mg, 0.2 mmol) at room temperature, and the mixture was stirred with heating at 40°C for 1 hour. After the temperature was decreased to room temperature, a sodium bicarbonate aqueous solution (100 mg) was added to stop the reaction, and the mixture was concentrated under reduced pressure. The concentrate was purified by chromatography (eluent: dichloromethane:methanol 32:1 → 16:1) to obtain a desired product (35.8 mg, 47%) as a white solid.
¹H-NMR (DMSO-d₆)δ1.83 (s, 3H), 2.17 (s, 3H), 4.00-4.03 (m, 2H), 4.69 (dt, J=23.8, 5.8 Hz, 1H), 5.31 (dd, J=21.7, 6.7 Hz, 1H), 5.59 (d, J=50.7 Hz, 1H), 6.66 (d, J=6.7 Hz, 1H).
ESI MASS m/z 383.8 (M+H)⁺

### Example 7. Synthesis of N²-dimethylaminomethylene-8,2'-anhydro-8-mercapto-9-β-arabinofuranosylguanine.

N,N-dimethylformamide dimethylacetal (1.0 mL, 7.5 mmol) was added to an N,N-dimethylformamide suspension (10 mL) of 8,2'-anhydro-8-mercapto-9-β-arabinofuranosylguanine (594.6 mg, 2 mmol) synthesized according to the method described in Can. J. Chem., vol. 50, p. 1100 (1972), and the mixture was stirred at room temperature for 13 hours. The resulting reaction solution was concentrated under reduced pressure. The concentrate was purified by chromatography (eluent: dichloromethane:methanol 8:1) to obtain a desired product (728.0 mg, 92%) as a white solid.
¹H-NMR (DMSO-d₆)δ3.03 (s, 3H), 3.16 (s, 3H), 3.35-3.41 (m, 1H), 3.46-3.51 (m, 1H), 3.91-3.95 (m, 1H), 4.33-4.37 (m, 1H), 4.81 (dd, J=6.6, 2.3 Hz, 1H), 4.92 (t, J=5.5 Hz, 1H), 5.87 (d, J=4.6 Hz, 1H), 6.34 (d, J=6.6 Hz, 1H), 8.51 (s, 1H).
ESI MASS m/z 352.6 (M+H)⁺

### Example 8. Synthesis of N²-dimethylaminomethylene-5'-O-trityl-8,2'-anhydro-8-mercapto-9- β-arabinofuranosylguanine.

Trityl chloride (669 mg, 2.4 mmol) was added to an N,N-dimethylformamide (2 mL)/pyridine (2 mL) mixed solution of N²-dimethylaminomethylene-8,2'-anhydro-8-mercapto-9-β-arabinofuranosylguanine (710 mg, 1.8 mmol) in three divided portions at intervals of 10 minutes, and the mixture was stirred at room temperature for 25 hours. The resulting reaction solution was concentrated under reduced pressure. The concentrate was purified by chromatography (eluent: dichloromethane:methanol 8:1) to obtain a desired product (558.8 mg, 52%) as a white solid.
¹H-NMR (DMSO-d₆)δ2.98-3.02 (m, 1H), 3.02 (s, 3H), 3.08-3.14 (m, 1H), 3.17 (s, 3H), 4.12-4.17 (m, 1H), 4.37-4.41 (m, 1H), 4.76 (dd, J=6.4, 3.3 Hz, 1H), 5.91 (d, J=4.7 Hz, 1H), 6.44 (d, J=6.4 Hz, 1H), 7.10-7.30 (m, 15H), 8.56 (s, 1H).
ESI MASS m/z 595.0 (M+H)⁺

### Example 9. Synthesis of N²-dimethylaminomethylene-5'-O-trityl-8,2'-anhydro-3'-deoxy-3'-fluoro-8-mercapto-9-β-arabinofuranosylguanine.

Diethylaminosulfur trifluoride (0.05 mL, 0.1 mmol) was added to a dichloromethane (1.0 mL) suspension of N²-dimethylaminomethylene-5'-O-trityl-8,2'-anhydro-8-mercapto-9-β-arabinofuranosylguanine (59.5 mg, 0.1 mmol) at room temperature, and the mixture was stirred with heating at 40°C for 1 hour. The resulting reaction solution was diluted with dichloromethane (4 ml), and the diluted solution was added to a mixed solution of a saturated sodium bicarbonate aqueous solution (1.5 mL) and water (1.5 mL) to stop the reaction. The solution was separated into layers. Dichloromethane (5 mL) was added to the aqueous layer and the mixture was re-extracted. The resulting two organic layers were mixed, and the mixture was dried over anhydrous magnesium sulfate and concentrated under reduced pressure. The concentrate was purified by chromatography (eluent: dichloromethane:methanol 32:1 → 16:1) to obtain a desired product (26.0 mg, 44%) as a white solid.
¹H-NMR (DMSO-d₆)δ2.96-3.20 (m, 2H), 3.03 (s, 3H), 3.17 (s, 3H), 4.55 (dt, J=24.9, 6.5 Hz, 1H), 5.24 (dd, J=20.2, 6.4 Hz, 1H), 5.52 (d, J=50.9 Hz, 1H), 6.53 (d, J=6.4 Hz, 1H), 7.15-7.30 (m, 15H), 8.55 (s, 1H), 11.40 (br, 1H).
ESI MASS m/z 597.0 (M+H)⁺

The present invention provides methods of producing purine nucleoside derivatives which are fluorinated at the 3'-position (preferably the α-position), and further provides intermediate compounds useful for the production of such derivatives and production methods thereof. The present invention provides an economic and efficient method of producing purine nucleoside derivatives which are fluorinated at the 3'- α-position, which is suitable as an industrial production method, as well as an intermediates therefor and a production methods thereof.

## Claims

1. A method of producing a fluorinated purine nucleoside represented by formula (2): 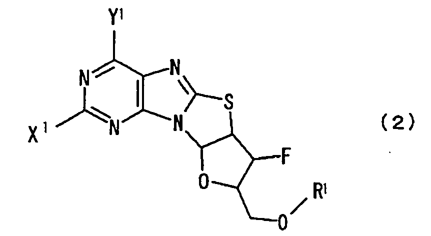 -wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, which comprises reacting a purine nucleoside represented by formula (1): 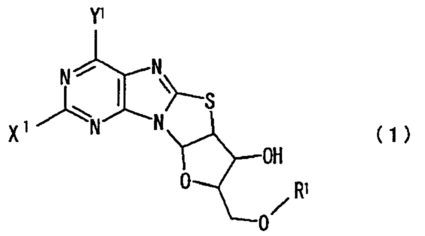 wherein R¹, X¹, and Y¹ are as defined above, with a fluorinating agent.

2. A method of producing a fluorinated purine nucleoside represented by formula (3): 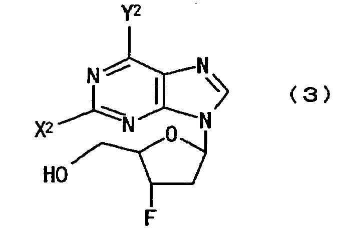 wherein X² is a hydrogen atom, a halogen atom, an optionally substituted amino group, or an optionally substituted hydroxyl group; and Y² is a hydrogen atom, a halogen atom, an optionally substituted amino group, or an optionally substituted hydroxyl group, which comprises subjecting a fluorinated purine nucleoside represented by formula (2): 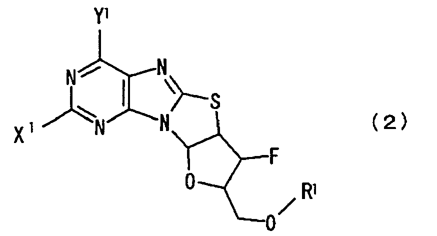 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, to, in any order:
(A) desulfurizing to remove sulfur atom,
(B) deprotecting to eliminate R¹, and
(C) when X¹ and X² are different groups, and/or Y¹ and Y² are different groups, converting X¹ to X² and/or Y¹ to Y².

3. A method of producing a fluorinated purine nucleoside represented by formula (3): 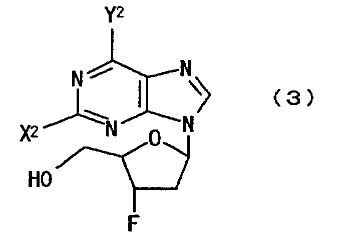 wherein X² is a hydrogen atom, a halogen atom, an optionally substituted amino group, or an optionally substituted hydroxyl group; and Y² is a hydrogen atom, a halogen atom, an optionally substituted amino group, or an optionally substituted hydroxyl group, which comprises reacting a purine nucleoside represented by formula (1): 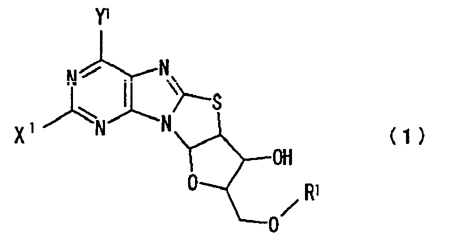 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, with a fluorinating agent to obtain a fluorinated purine nucleoside represented by formula (2): 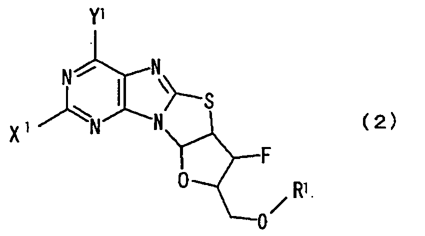 wherein R¹, X¹, and Y¹ are as defined above, and then subjecting the fluorinated purine nucleoside to, in any order:
(A) desulfurizing to remove sulfur atom,
(B) deprotecting to eliminate R¹, and
(C) when X¹ and X² are different groups, and/or Y¹ and Y² are different groups, converting X¹ to X² and/or Y¹ to Y².

4. A method of producing a purine nucleoside represented by formula (1): 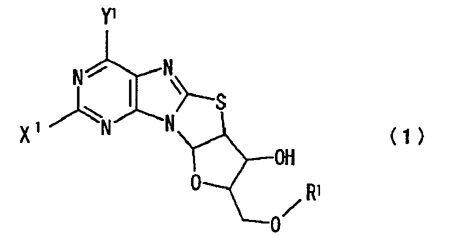 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, which comprises subjecting a purine nucleoside represented by formula (A): 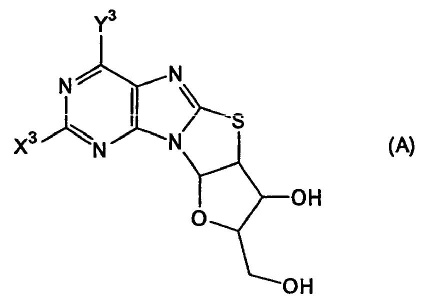 wherein X³ is a hydrogen atom, an amino group, or a hydroxyl group; and Y³ is a hydrogen atom, an amino group, or a hydroxyl group, to, in any order:
(D) introducing R¹ into the 5'-position hydroxyl group to protect the hydroxyl group, and
(E) when X³ and X¹ are different groups, and/or when Y³ and Y¹ are different groups, converting X³ to X¹ and/or Y³ to Y¹.

5. A method of producing a fluorinated purine nucleoside represented by formula (3): 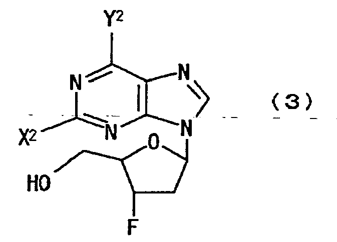 wherein X² is a hydrogen atom, a halogen atom, an optionally substituted amino group, or an optionally substituted hydroxyl group; and Y² is a hydrogen atom, a halogen atom, an optionally substituted amino group, or an optionally substituted hydroxyl group, which comprises subjecting a purine nucleoside represented by formula (A): 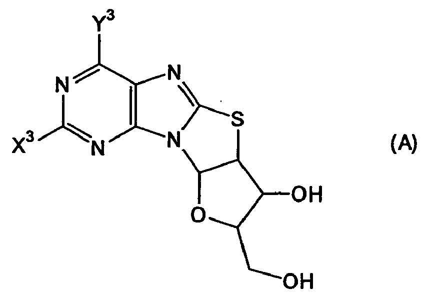 wherein X³ is a hydrogen atom, an amino group, or a hydroxyl group; and Y³ is a hydrogen atom, an amino group, or a hydroxyl group, to, in any order:
(D) introducing R¹ into the 5'-position hydroxyl group to protect the hydroxyl group, and
(E) when X³ and X¹ are different groups, and/or when Y³ and Y¹ are different groups, converting X³ to X¹ and/or Y³ to Y¹, to obtain a purine nucleoside represented by formula (1): 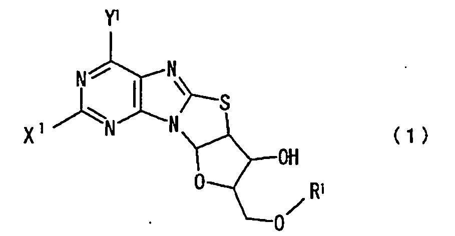 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group, reacting the purine nucleoside with a fluorinating agent to obtain a fluorinated purine nucleoside represented by formula (2): 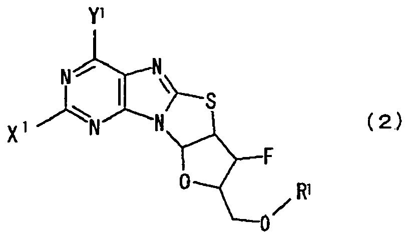 wherein R¹, X¹, and Y¹ are as defined above, and then subjecting the fluorinated purine nucleoside to, in any order:
(A) desulfurizing to remove sulfur atom,
(B) deprotecting to eliminate R¹, and
(C) when X¹ and X² are different groups, and/or Y¹ and Y² are different groups, converting X¹ to X² and/or Y¹ to Y².

6. The method of any one of claims 1, 3 and 5, wherein the fluorinating agent is a perfluoroalkanesulfonyl fluoride and the reacting with a fluorinating agent is carried out in the presence of a base.

7. The method of any one of claims 1 and 3 to 5, wherein the purine nucleoside represented by formula (1) has a relative configuration represented by formula (1'): 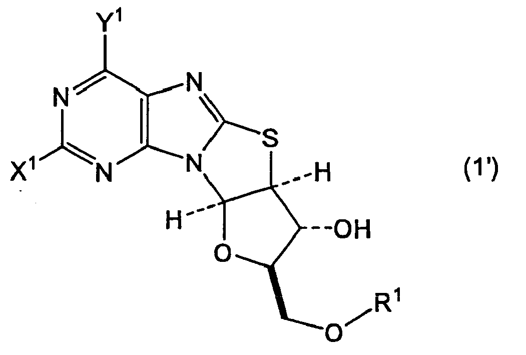 wherein R¹, X¹, and Y¹ are as defined in claim 1.

8. The method of any one of claims 1 to 3 and 5, wherein the fluorinated purine nucleoside represented by formula (2) has a relative configuration represented by formula (2'): 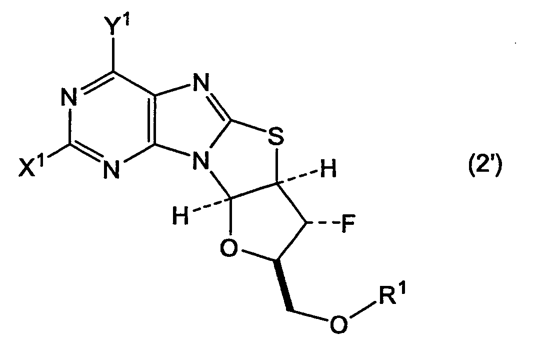 wherein R¹, X¹, and Y¹ are as defined in claim 1.

9. The method of any one of claims 2, 3 and 5, wherein the fluorinated purine nucleoside represented by formula (3) has a relative configuration represented by formula (3'): 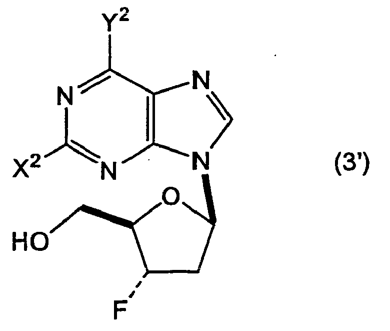 wherein X² and Y² are as defined in claim 2.

10. The method of claim 4 or 5, wherein the purine nucleoside represented by formula (A) has a relative configuration represented by formula (A'): 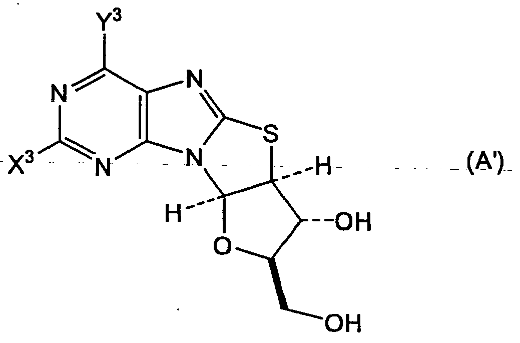 wherein X³ and Y³ are as defined in claim 4.

11. A method of producing a fluorinated purine nucleoside represented by formula (5): 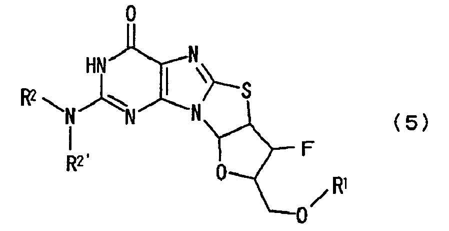 wherein R¹ is a hydroxyl-protecting group; and R² and R^{2'} are each independently a hydrogen atom or an amino-protecting group, or R² and R^{2'} in combination optionally form an amino-protecting group, which comprises reacting a purine nucleoside represented by formula (4): 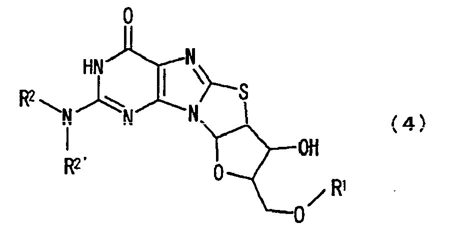 wherein R¹, R², and R^{2'} are as defined above, with a fluorinating agent.

12. A method of producing a fluorinated purine nucleoside represented by formula (6): 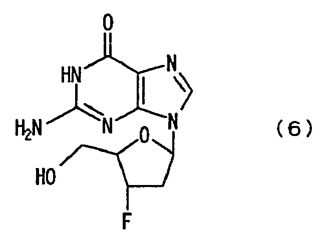 which comprises subjecting a fluorinated purine nucleoside represented by formula (5): 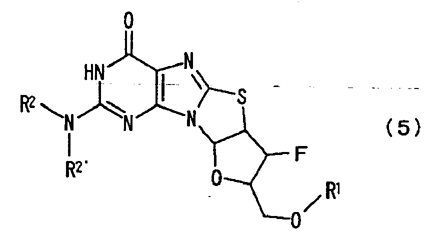 wherein R¹ is a hydroxyl-protecting group; and R² and R^{2'} are each independently a hydrogen atom or an amino-protecting group, or R² and R^{2'} in combination optionally form an amino-protecting group, to, in any order:
(A) desulfurizing to remove sulfur atom,
(B) deprotecting to eliminate R¹, and
(C') when R² and/or R^{2'} are/is amino-protecting group(s), deprotecting to eliminate the protecting group.

13. A method of producing a fluorinated purine nucleoside represented by formula (6): 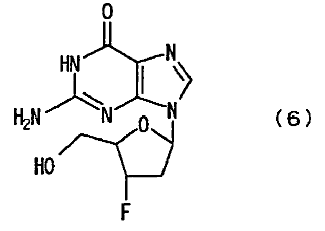 which comprises reacting a purine nucleoside represented by formula (4): 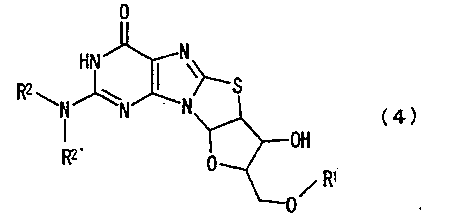 wherein R¹ is a hydroxyl-protecting group; and R² and R^{2'} are each independently a hydrogen atom or an amino-protecting group, or R² and R^{2'} in combination optionally form an amino-protecting group, with a fluorinating agent to obtain a fluorinated purine nucleoside represented by formula (5): 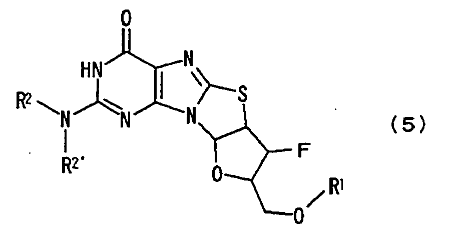 wherein R¹, R², and R^{2'} are as defined above, and subjecting the fluorinated purine nucleoside to, in any order:
(A) desulfurizing to remove sulfur atom,
(B) deprotecting to eliminate R¹, and
(C') when R² and/or R^{2'} are/is amino-protecting group(s), deprotecting to eliminate the protecting group.

14. A method of producing a purine nucleoside represented by formula (4): 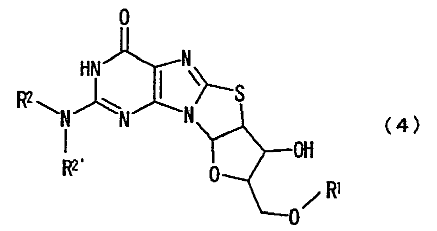 wherein R¹ is a hydroxyl-protecting group; and R² and R^{2'} are each independently a hydrogen atom or an amino-protecting group, or R² and R^{2'} in combination optionally form an amino-protecting group, which comprises subjecting a purine nucleoside represented by formula (B): 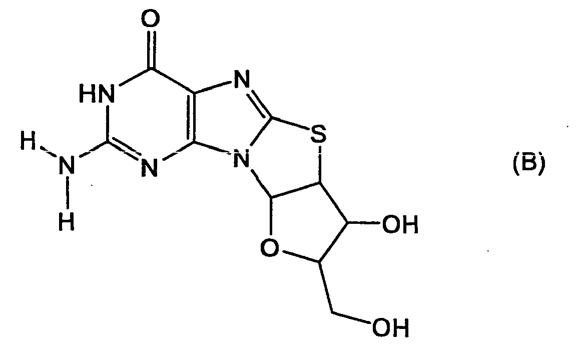 to, in any order:
(D) introducing R¹ into the 5'-position hydroxyl group to protect the hydroxyl group, and
(E') when R² and/or R^{2'} are/is amino-protecting group(s), introducing the protecting group.

15. A method of producing a fluorinated purine nucleoside represented by formula (6): 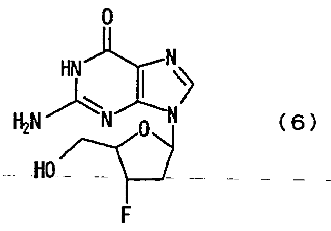 which comprises subjecting a purine nucleoside represented by formula (B): 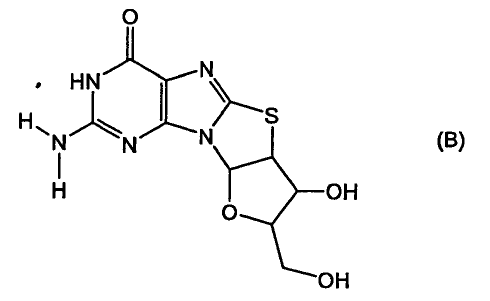 to, in any order:
(D) introducing R¹ into the 5'-position hydroxyl group to protect the hydroxyl group, and
(E') when R² and/or R^{2'} are/is amino-protecting group(s), introducing the protecting group, to obtain a purine nucleoside represented by formula (4): 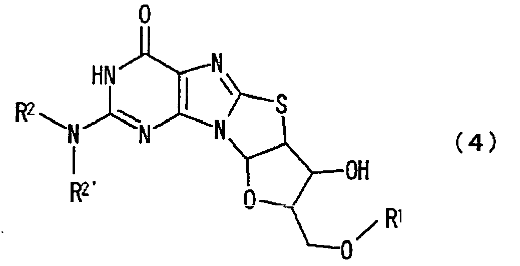 wherein R¹ is a hydroxyl-protecting group; and R² and R^{2'} are each independently a hydrogen atom or an amino-protecting group, or R² and R^{2'} in combination optionally form an amino-protecting group, reacting the purine nucleoside with a fluorinating agent to obtain a fluorinated purine nucleoside represented by formula (5): 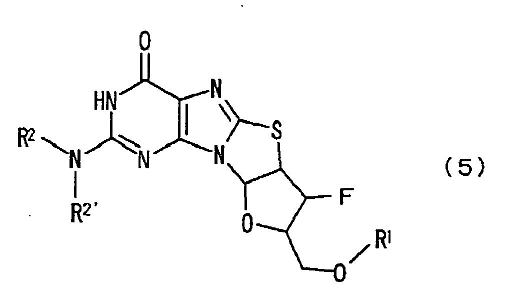 wherein R¹, R², and R^{2'} are as defined above, and then subjecting the fluorinated purine nucleoside to, in any order:
(A) desulfurizing to remove sulfur atom,
(B) deprotecting to eliminate R¹, and
(C') when R² and/or R^{2'} are/is amino-protecting group(s), deprotecting to eliminate the protecting group.

16. The method of any one of claims 11, 13 and 15, wherein the fluorinating agent is perfluoroalkanesulfonyl fluoride and the reacting with a fluorinating agent is carried out in the presence of a base.

17. The method of any one of claims 11 and 13 to 15, wherein the purine nucleoside represented by formula (4) has a relative configuration represented by formula (4'): 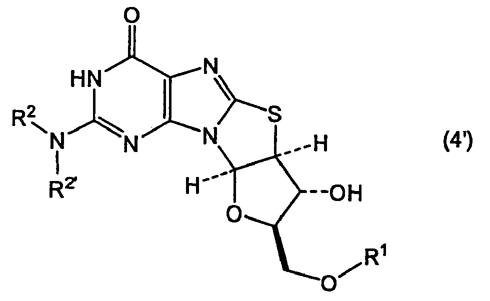 wherein R¹, R², and R^{2'} are as defined in claim 11.

18. The method of any one of claims 11 to 13 and 15, wherein the fluorinated purine nucleoside represented by formula (5) has a relative configuration represented by formula (5'): 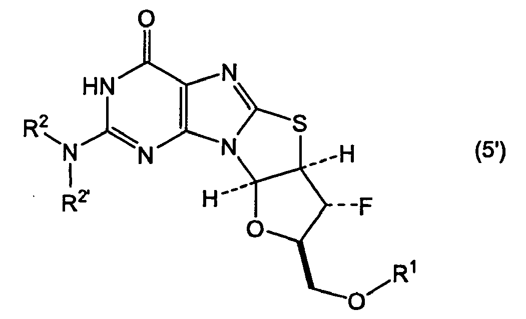 wherein R¹, R², and R^{2'} are as defined in claim 11.

19. The method of any one of claims 12, 13 and 15, wherein the fluorinated purine nucleoside represented by formula (6) has a relative configuration represented by formula (6'):

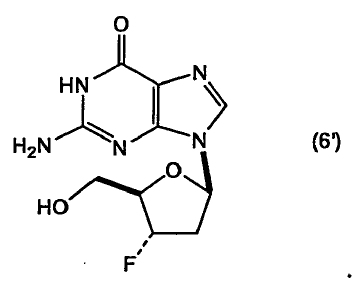

20. The method of claim 14 or 15, wherein the purine nucleoside represented by formula (B) has a relative configuration represented by formula (B'):

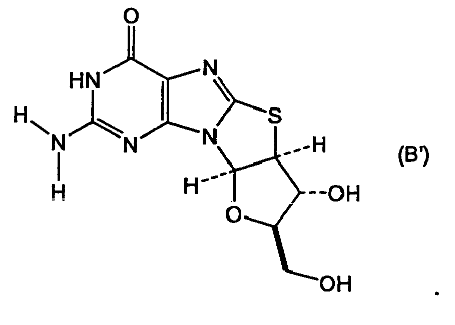

21. The method of any one of claims 11 to 15, wherein R¹ and R² are each a trityl group, a 9-phenylfluoren-9-yl group, or an acetyl group, each optionally having one or more substituents, and R^{2'} is a hydrogen atom.

22. The method of any one of claims 11 to 15, wherein R¹ is a trityl group optionally having one or more substituents, and R² and R^{2'} in combination form a dimethylaminomethylidene group.

23. A 8,2'-anhydro-8-mercapto-9-arabinofuranosyl purine represented by formula (1): 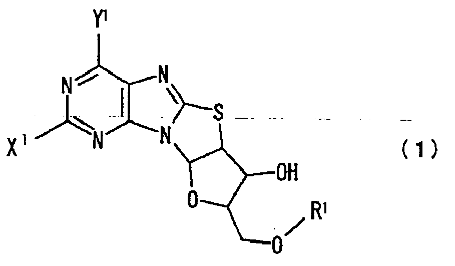 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group.

24. The 8,2'-anhydro-8-mercapto-9-arabinofuranosyl purine of claim 23, which has a relative configuration represented by formula (1'): 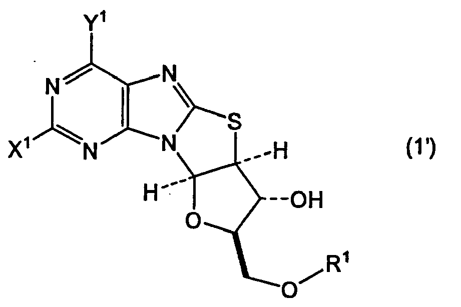 wherein R¹, X¹, and Y¹ are as defined in claim 23.

25. A 8,2'-anhydro-8-mercapto-9-arabinofuranosyl guanine represented by formula (4): 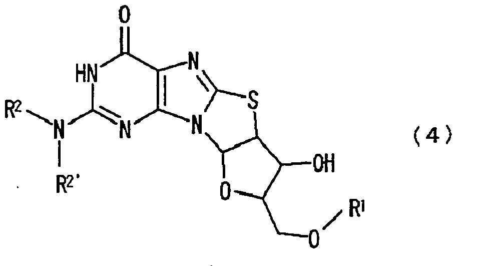 wherein R¹ is a hydroxyl-protecting group; and R² and R^{2'} are each independently a hydrogen atom or an amino-protecting group, or R² and R^{2'} in combination optionally form an amino-protecting group.

26. The 8,2'-anhydro-8-mercapto-9-arabinofuranosyl guanine of claim 25, which has a relative configuration represented by formula (4'): 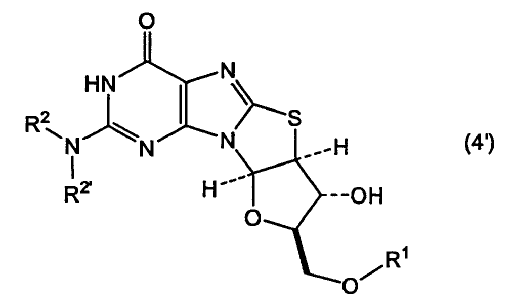 wherein R¹, R², and R^{2'} are as defined in claim 25.

27. The 8,2'-anhydro-8-mercapto-9-arabinofuranosyl guanine of claim 25 or 26, wherein R¹ and R² are each a trityl group, a 9-phenylfluoren-9-yl group, or an acetyl group, each optionally having one or more substituents, and R^{2'} is a hydrogen atom.

28. The 8,2'-anhydro-8-mercapto-9-arabinofuranosyl guanine of claim 25 or 26, wherein R¹ is a trityl group optionally having one or more substituents, and R² and R^{2'} in combination form a dimethylaminomethylidene group.

29. A 8,2'-anhydro-3'-deoxy-3'-fluoro-8-mercapto-9-arabinofuranosyl purine represented by formula (2): 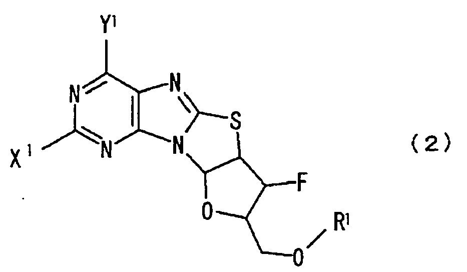 wherein R¹ is a hydroxyl-protecting group; X¹ is a hydrogen atom, a halogen atom, an optionally substituted amino group, or an optionally substituted hydroxyl group; and Y¹ is a hydrogen atom, a halogen atom, an optionally substituted amino group, or an optionally substituted hydroxyl group.

30. The 8,2'-anhydro-3'-deoxy-3'-fluoro-8-mercapto-9-arabinofuranosyl purine of claim 29, which has a relative configuration represented by formula (2'): 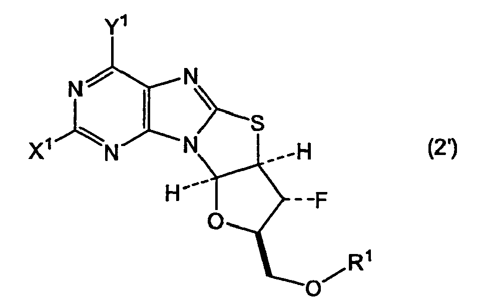 wherein R¹, X¹, and Y¹ are as defined in claim 29.

31. A 8,2'-anhydro-3'-deoxy-3'-fluoro-8-mercapto-9-arabinofuranosyl guanine represented by formula (5): 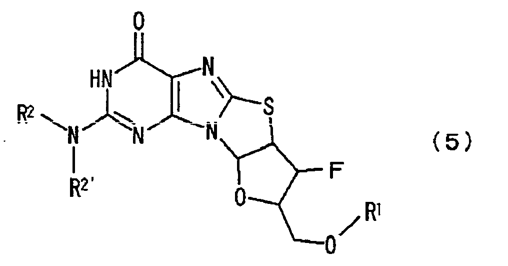 wherein R¹ is a hydroxyl-protecting group; and R² and R²' are each independently a hydrogen atom or an amino-protecting group, or R² and R²' in combination optionally form an amino-protecting group.

32. The 8,2'-anhydro-3'-deoxy-3'-fluoro-8-mercapto-9-arabinofuranosyl guanine of claim 31, which has a relative configuration represented by formula (5'): 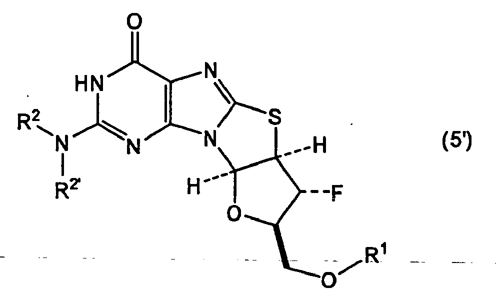 wherein R¹, R², and R^{2'} are as defined in claim 31.

33. The 8,2'-anhydro-3'-deoxy-3'-fluoro-8-mercapto-9-arabinofuranosyl guanine of claim 31 or 32, wherein R¹ and R² are each a trityl group, a 9-phenylfluoren-9-yl group or an acetyl group, each optionally having one or more substituents, and R^{2'} is a hydrogen atom.

34. The 8,2'-anhydro-3'-deoxy-3'-fluoro-8-mercapto-9-arabinofuranosyl guanine of claim 31 or 32, wherein R¹ is a trityl group optionally having one or more substituents, and R² and R^{2'} in combination form a dimethylaminomethylidene group.
